# EUROPÄISCHE PATENTSCHRIFT

(11) **EP 1 282 700 B1**
(45) Veröffentlichungstag und Bekanntmachung des Hinweises auf die Patenterteilung: **27.02.2008**
(21) Anmeldenummer: 01936370.4
(22) Anmeldetag: 17.05.2001
(51) Int. Cl.: C12N 15/11, C12N 15/54, C12N 5/10, C12N 9/12, C12Q 1/68, C07K 16/40, G01N 33/577, A01K 67/027, A61K 31/713, A61K 38/43, A61K 39/395

(54) **NEURONALE SERIN-THREONIN-PROTEIN-KINASE**
NEURONAL SERINE-THREONINE PROTEIN KINASE
SERINE-THREONINE PROTEINE KINASE NEURONALE

(30) Priorität: 17.05.2000 DE 10024171
(43) Veröffentlichungstag der Anmeldung: 12.02.2003
(73) Patentinhaber: AXARON Bioscience AG, 69120 Heidelberg (DE)
(72) Erfinder: SCHNEIDER, Armin, 69118 Heidelberg (DE); KLAUSSNER, Bettina, 69123 Heidelberg (DE); FISCHER, Achim, 69120 Heidelberg (DE); NEWRZELLA, Dieter, 69221 Dossenheim (DE); GÖTZ, Bernhard, 69121 Heidelberg (DE); ROSSNER, Moritz, 68723 Schwetzingen (DE); EISENHARDT, Gisela, 69126 Heidelberg (DE); KUNER, Rohini, 69126 Heidelberg (DE); TRUTZEL, Annette, 67227 Frankenthal (DE); KAMMANDEL, Birgitta, 69115 Heidelberg (DE); JOMANA NAIM, Stephanie, 69123 Heidelberg (DE); SCHWANINGER, Markus, 69120 Heidelberg (DE)
(74) Vertreter: Isenbruck, Günter
(86) Internationale Anmeldenummer: PCT/EP2001/005660
(87) Internationale Veröffentlichungsnummer: WO 2001/088108

(56) Entgegenhaltungen:
- EP-A- 1 130 094
- WO-A-97/23625
- WO-A-98/01756
- WO-A-99/42592
- WO-A-02/064791
- DATABASE EMBL [Online] Hinxton, GB; Accession: AF240782, 20. April 2000 (2000-04-20) DARMON, Y ET AL: "Mus musculus ELKL motif Kinase 2 long form (EMK2) mRNA, complete cds." XP002175126
- DATABASE EMBL [Online] Hinxton ,GB; Accession No: U64205, 28. April 1998 (1998-04-28) PENG, C.Y ET AL: "Homo sapiens Cdc25C associated protein kinase C-TAK1 mRNA, complete cds. " XP002175127 -& PENG CHENG-YUAN ET AL: "C-TAK1 protein kinase phosphorylates human Cdc25C on serine 216 and promotes 14-3-3 protein binding." CELL GROWTH & DIFFERENTIATION, Bd. 9, Nr. 3, März 1998 (1998-03), Seiten 107-208, XP001015918
- DATABASE EMBL [Online] Hinxton; Accession No: AC005781, 8. Oktober 1998 (1998-10-08) LAMERDIN, J.E. ET AL: "Homo sapiens chromosome 19, cosmid R33632, complete sequence." XP002175128
- DATABASE EMBL [Online] Hinxton, GB; Accesion: AB058763, 10. Mai 2001 (2001-05-10) OHARA, O ET AL: "Homo sapiens mRNA for KIAA1860 protein, partial cds" XP002175129
- DATABASE EMBL [Online] Hinxton, GB; Accession No: AK027619, 15. Mai 2001 (2001-05-15) ISOGAI, T ET AL: "Homo sapiens cDNA FLJ14713 fis, clone NT2RP3000845, moderately similar to putative Serine-Threonine-Protein Kinase P78 (EC 2.7.1.-)" XP002175130
- DATABASE EMBL [Online] Hinxton, GB; Accession No: AB049127, 19. März 2001 (2001-03-19) NAKAMURA, Y. ET AL: "Homo sapiens MARKL1 mRNA for MAP/microtubule affinity regulating kinase like 1, complete cds" XP002175131 & KATO TATSUSHI ET AL: "Isolation of a novel human gene, MARKL1, homologous to MARK3 and its involvement in hepatocellular carcinogenesis." NEOPLASIA (NEW YORK), Bd. 3, Nr. 1, Januar 2001 (2001-01), Seiten 4-9, XP002175125
- DREWES G ET AL: "MARK, a novel family of protein kinases that phosphorylate microtubule-associated proteins and trigger microtubule disruption" CELL, CELL PRESS, CAMBRIDGE, NA, US, Bd. 89, 18. April 1997 (1997-04-18), Seiten 297-308, XP002105681 ISSN: 0092-8674

### SEQUENZPROTOKOLL

<110> BASF-LYNX Bioscience
<120> Neuronale Serin-Threonin-Proteinkinase
<130> p31560
<140>
   <141>
<160> 8
<170> PatentIn Ver. 2.1
<210> 1
   <211> 3170
   <212> DNA
   <213> Mus musculus
<220>
   <221> polyA_signal
   <222> (3094)..(3099)
<220>
   <223> Kodierende Sequenz: (1)...(2172)
<400> 1
<210> 2
   <211> 3250
   <212> DNA
   <213> Mus musculus
<220>
   <221> polyA__signal
   <222> (3174)..(3179)
<220>
   <223> Kodierende Sequenz: (1)...(1980)
<400> 2
   aaagggccgt cctggtccag ccgttccctg ggtgcccgtt gccggaactc tatcgcttcc 60
   tgccctgagg aacaacccca tgtgggcaac tataggctgc taaggaccat cgggaagggc 120
   aacttcgcca aagtcaagct ggctcggcat atcctcacgg gccgggaggt cgctattaag 180
   atcattgata agacccagct gaaccccagt agcttgcaga agctgttcag agaagtccga 240
   attatgaagg gactcaacca ccccaacatc gtgaagcttt ttgaggtgat agagacggag 300
   aagacgctat acctggtgat ggaatacgct agcgcaggag aagtgtttga ctacctcgtg 360
   tcgcacggcc gcatgaagga gaaggaggct cgagccaagt tgcggcagat cgtgtcagcc 420
   gtgcactact gtcatcagaa gaacattgta cacagggatc taaaggctga aaacctgttg 480
   ctggatgccg aggccaacat caaaatcgcc gacttcggct tcagcaatga gttcacgctg 540
   ggctccaagc tggacacctt ctgtgggagc cccccatacg ccgccccaga gctgttccag 600
   ggcaagaagt atgatgggcc agaggtggac atctggagcc tgggtgtcat cctgtacacg 660
   ctggtcagcg gctccctgcc cttcgatggg cacaacctca aggagctgcg ggagcgaatc 720
   ctcagaggaa agtaccgggt ccccttctac atgtctacag actgcgagag cattctgcgg 780
   agatttctgg tgctgaaccc cgcaaaacgc tgtactctgg agcaaatcat gaaagacaaa 840
   tggatcaaca tcggctatga gggtgaggag ctgaagccat acacggagcc tgaggaggac 900
   ttcggggaca ccaagagaat tgaggtgatg gtgggtatgg gctacacacg ggaagaaatc 960
   aaagaggcct tgaccaacca gaagtacaac gaggtgaccg ccacctacct cctgctgggc 1020
   aggaagactg atgagggtgg ggaccggggt gccccagggc tggccctggc acgggtgcgg 1080
   gcgcccagcg acaccaccaa cgggacaagc tccagcaaag gcagcagcca caacaaaggg 1140
   caacgggctt cttcctccac ctaccaccgc cagcgccgtc acagtgactt ctgtggcccg 1200
   tcccctgccc cgctgcaccc gaagcgcagc ccaaccagca cgggagacac ggagctcaaa 1260
   gaagagcgga tgccgggtcg gaaagcgagc tgcagtgcag tgggcagtgg aagtcgaggc 1320
   ttgcccccct ccagccccat ggtcagcagt gcccacaacc ccaataaggc agagatccct 1380
   gagcggcgga aggacagcac tagcacccct aacaacctcc cccccagcat gatgacccga 1440
   agaaacacct atgtgtgcac agagcgacca ggatctgaac gcccgtcctt gttgccaaat 1500
   ggcaaagaaa atagctccgg tacctcgcgg gtgccccctg cctcgccttc cagtcatagc 1560
   ctggctcccc cgtcaggcga gcggagccgc ctggctcggg gctccaccat ccgcagcacc 1620
   ttccatgggg gccaggtccg agaccggcgg gcagggagcg ggagtggcgg gggtgtgcag 1680
   aatggacccc cagcctcacc cacgcttgcc cacgaggccg cacccctgcc ctccgggcgg 1740
   cctcgcccca ccaccaacct cttcaccaag ctgacctcca aactgacccg aagggttacc 1800
   ctcgatccct ctaaacggca gaactctaac cgctgtgtct cgggcgcctc tctgccccag 1860
   ggatccaaaa tcaggtcaca gacgaacctg agagaatcgg gggacctgag gtcacaagtt 1920
   gccatctacc ttgggataaa acggaaaccg cccccaggct gctccgattc ccctggagtg 1980
   tgaagctgac cagctcgcga cctcctgagg ccctgatggc tgccatgcga caggccacag 2040
   cggccgcccg ctgccggtgc cgccagccgc agccgttcct gctggcctgc ctgcacgggg 2100
   gtgcgggcgg gcccgagccc ctgtcccatt tcgaagtgga ggtgtgccag ctgccccggc 2160
   ccggcctcag gggcgtcctc ttccgccgtg tggcgggcac cgccctggcc ttccgcaccc 2220
   tcgtcacccg catctccaac gacctcgaac tctgagccac cgccaccact accaccgcca 2280
   cagccaccat cacagcccgg gtcccttctt tctctggttc ctttcacttc cccaagaggg 2340
   gaagaggaca gaggagaggg tgccctgtgt catgactgaa gtttccctgg attagattgg 2400
   tggacagaga cagtgtgggg acacatgaca tgataagagg gctcagcagg gggagctggc 2460
   accctcctag ggcctctggt gggacccccc tccccacaat cttgttcttc tgcagggcac 2520
   ctgaggagac tttggggaca ggagtgagaa gggaaactga ggaaattctc ccattcaggg 2580
   agagctgcca ggattaatga ctggagacag acttgggggg ttcagggagt tgggggagtc 2640
   acagacagaa accttcccct cactccccct tatgatcgaa cctccttcat gccccaggct 2700
   ggcgcggggc actttgtaca aatccgtgta tatactcctg tccctctgca gaggtctctc 2760
   ggggagctgc tgctgccgcc tccgattttt aagttattgc cccgcccctt ctgtcagctc 2820
   ctcatctgca gcctgttact caataaacag taggagtccc tccaaccccg acctcctccc 2880
   tggccgacct ggggtttccc ttctcagccc ttggcctgca ggtgagccag ggagctgggg 2940
   acttgacccc aacctgtggt tctgcttgct gagcctttgt tatctcatct tcagaatggg 3000
   aacagtgggg ttggaggatg ggtcaaggat gactatggaa gagggcagaa cagagctcag 3060
   cctcttccac gaggccccag ccttctgtga caccctcctc ttggccactc actcccctct 3120
   gccatattac actggaccca gagcctcttc ctattccagt aatacatgta ttcaataaac 3180
   aatcaacgac tggtgccgac tccacgctag gcccagttct ggacataaaa aaaaaaaaaa 3240
   aaaaaaaaaa                                                        3250
<210> 3
   <211> 3312
   <212> DNA
   <213> Homo sapiens
<220>
   <221> polyA_signal
   <222> (3275)..(3280)
<220>
   <223> Kodierende Sequenz: (64)...(2319)
<400> 3
   aggggaccct gggacccccg ccccccccac ccggccgccc ctgccccccg ggacccggag 60
   aagatgtctt cgcggacggt gctggccccg ggcaacgatc ggaactcgga cacgcatggc 120
   accttgggca gtggccgctc ctcggacaaa ggcccgtcct ggtccagccg ctcactgggt 180
   gcccgttgcc ggaactccat cgcctcctgt cccgaggagc agccccacgt gggcaactac 240
   cgcctgctga ggaccattgg gaagggcaac tttgccaaag tcaagctggc tcggcacatc 300
   ctcactggtc gggaggttgc catcaagatt atcgacaaaa cccagctgaa tcccagcagc 360
   ctgcagaagc tgttccgaga agtccgcatc atgaagggcc taaaccaccc caacatcgtg 420
   aagctctttg aggtgattga gactgagaag acgctgtacc tggtgatgga gtacgcaagt 480
   gctggagaag tgtttgacta cctcgtgtcg catggccgca tgaaggagaa ggaagctcga 540
   gccaagttcc gacagattgt ttcggctgtg cactattgtc accagaaaaa tattgtacac 600
   agggacctga aggctgagaa cctcttgctg gatgccgagg ccaacatcaa gattgctgac 660
   tttggcttca gcaacgagtt cacgctggga tcgaagctgg acacgttctg cgggagcccc 720
   ccatatgccg ccccggagct gtttcagggc aagaagtacg acgggccgga ggtggacatc 780
   tggagcctgg gagtcatcct gtacaccctc gtcagcggct ccctgccctt cgacgggcac 840
   aacctcaagg agctgcggga gcgagtactc agagggaagt accgggtccc tttctacatg 900
   tcaacagact gtgagagcat cctgcggaga tttttggtgc tgaacccagc taaacgctgt 960
   actctcgagc aaatcatgaa agacaaatgg atcaacatcg gctatgaggg tgaggagttg 1020
   aagccataca cagagcccga ggaggacttc ggggacacca agagaattga ggtgatggtg 1080
   ggtatgggct acacacggga agaaatcaaa gagtccttga ccagccagaa gtacaacgaa 1140
   gtgaccgcca cctacctcct gctgggcagg aagactgagg agggtgggga ccggggcgcc 1200
   ccagggctgg ccctggcacg ggtgcgggcg cccagcgaca ccaccaacgg aacaagttcc 1260
   agcaaaggca ccagccacag caaagggcag cggagttcct cttccaccta ccaccgccag 1320
   cgcaggcata gcgatttctg tggcccatcc cctgcacccc tgcaccccaa acgcagcccg 1380
   acgagcacgg gggaggcgga gctgaaggag gagcggctgc caggccggaa ggcgagctgc 1440
   agcaccgcgg ggagtgggag tcgagggctg cccccctcca gccccatggt cagcagcgcc 1500
   cacaacccca acaaggcaga gatcccagag cggcggaagg acagcacgag cacccccaac 1560
   aacctccctc ctagcatgat gacccgcaga aacacctacg tttgcacaga acgcccgggg 1620
   gctgagcgcc cgtcactgtt gccaaatggg aaagaaaaca gctcaggcac cccacgggtg 1680
   ccccctgcct ccccctccag tcacagcctg gcacccccat caggggagcg gagccgcctg 1740
   gcacgtggtt ccaccatccg cagcaccttc catggtggcc aggtccggga ccggcgggca 1800
   gggggtgggg gtggtggggg tgtgcagaat gggccccctg cctctcccac actggcccat 1860
   gaggctgcac ccctgcccgc cgggcggccc cgccccacca ccaacctctt caccaagctg 1920
   acctccaaac tgacccgaag ggtcgcagac gaacctgaga gaatcggggg acctgaggtc 1980
   acaagttgcc atctaccttg ggatcaaacg gaaaccgccc cccggctgct ccgattcccc 2040
   tggagtgtga agctgaccag ctcgcgccct cctgaggccc tgatggcagc tctgcgccag 2100
   gccacagcag ccgcccgctg ccgctgccgc cagccacagc cgttcctgct ggcctgcctg 2160
   cacgggggtg cgggcgggcc cgagcccctg tcccacttcg aagtggaggt ctgccagctg 2220
   ccccggccag gcttgcgggg agttctcttc cgccgtgtgg cgggcaccgc cctggccttc 2280
   cgcaccctcg tcacccgcat ctccaacgac ctcgagctct gagccaccac ggtcccaggg 2340
   cccttactct tcctctccct tgtcgccttc acttctacag gaggggaagg ggccagggag 2400
   gggattctcc ctttatcatc acctcagttt ccctgaatta tatttggggg caaagattgt 2460
   cccctctgct gttctctggg gccgctcagc acagaagaag gatgaggggg ctcagcgggg 2520
   ggagctggca ccttcctgga gcctccagcc agtcctgtcc tccctcgccc taccaagagg 2580
   gcacctgagg agactttggg gacagggcag gggcagggag ggaaactgag gaaatcttcc 2640
   attcctccca acagctcaaa attaggcctt gggcaggggc agggagagct gctgagccta 2700
   aagactggag aatctggggg actgggagtg ggggtcagag aggcagattc cttcccctcc 2760
   cgtcccctca cgctcaaacc cccacttcct gccccaggct ggcgcggggc actttgtaca 2820
   aatccttgta aataccccac accctcccct ctgcaaaggt ctcttgagga gctgccgctg 2880
   tcacctacgg tttttaagtt attacacccc gaccctcctc ctgtcagccc cctcacctgc 2940
   agcctgttgc ccaataaatt taggagagtc cccccctccc caatgctgac cctaggattt 3000
   tccttccctg ccctcacctg caaatgagtt aaagaagagg cgtgggaatc caggcagtgg 3060
   tttttccttt cggagcctcg gttttctcat ctgcagaatg ggagcggtgg gggtgggaag 3120
   gtaaggatgg tcgtggaaga aggcaggatg gaactcggcc tcatccccga ggccccagtt 3180
   cctatatcgg gccccccatt catccactca cactcccagc caccatgtta cactggactc 3240
   taagccactt cttactccag tagtaaattt attcaataaa caatcattga cccatgccta 3300
   aaaaaaaaaa aa                                                    3312
<210> 4
   <211> 3392
   <212> DNA
   <213> Homo sapiens
<220>
   <221> polyA_signal
   <222> (3355)..(3360)
<220>
   <223> Kodierende Sequenz: (64)...(2127)
<400> 4
<210> 5
   <211> 724
   <212> PRT
   <213> Mus musculus
<400> 5
   Lys Gly Pro Ser Trp Ser Ser Arg Ser Leu Gly Ala Arg Cys Arg Asn
   1               5                   10                  15
   Ser Ile Ala Ser Cys Pro Glu Glu Gln Pro His Val Gly Asn Tyr Arg
               20                  25                  30
   Leu Leu Arg Thr Ile Gly Lys Gly Asn Phe Ala Lys Val Lys Leu Ala
           35                  40                  45
   Arg His Ile Leu Thr Gly Arg Glu Val Ala Ile Lys Ile Ile Asp Lys
       50                  55                  60
   Thr Gln Leu Asn Pro Ser Ser Leu Gln Lys Leu Phe Arg Glu Val Arg
    65                 70                  75                  80
   Ile Met Lys Gly Leu Asn His Pro Asn Ile Val Lys Leu Phe Glu Val
                   85                  90                  95
   Ile Glu Thr Glu Lys Thr Leu Tyr Leu Val Met Glu Tyr Ala Ser Ala
               100                 105                 110
   Gly Glu Val Phe Asp Tyr Leu Val Ser His Gly Arg Met Lys Glu Lys
           115                 120                 125
   Glu Ala Arg Ala Lys Leu Arg Gln Ile Val Ser Ala Val His Tyr Cys
       130                 135                 140
   His Gln Lys Asn Ile Val His Arg Asp Leu Lys Ala Glu Asn Leu Leu
   145                 150                 155                 160
   Leu Asp Ala Glu Ala Asn Ile Lys Ile Ala Asp Phe Gly Phe Ser Asn
                   165                 170                 175
   Glu Phe Thr Leu Gly Ser Lys Leu Asp Thr Phe Cys Gly Ser Pro Pro
               180                 185                 190
   Tyr Ala Ala Pro Glu Leu Phe Gln Gly Lys Lys Tyr Asp Gly Pro Glu
           195                 200                 205
   Val Asp Ile Trp Ser Leu Gly Val Ile Leu Tyr Thr Leu Val Ser Gly
       210                 215                 220
   Ser Leu Pro Phe Asp Gly His Asn Leu Lys Glu Leu Arg Glu Arg Ile
   225                 230                 235                 240
   Leu Arg Gly Lys Tyr Arg Val Pro Phe Tyr Met Ser Thr Asp Cys Glu
                   245                 250                 255
   Ser Ile Leu Arg Arg Phe Leu Val Leu Asn Pro Ala Lys Arg Cys Thr
               260                 265                 270
   Leu Glu Gln Ile Met Lys Asp Lys Trp Ile Asn Ile Gly Tyr Glu Gly
           275                 280                 285
   Glu Glu Leu Lys Pro Tyr Thr Glu Pro Glu Glu Asp Phe Gly Asp Thr
       290                 295                 300
   Lys Arg Ile Glu Val Met Val Gly Met Gly Tyr Thr Arg Glu Glu Ile
   305                 310                 315                 320
   Lys Glu Ala Leu Thr Asn Gln Lys Tyr Asn Glu Val Thr Ala Thr Tyr
                   325                 330                 335
   Leu Leu Leu Gly Arg Lys Thr Asp Glu Gly Gly Asp Arg Gly Ala Pro
               340                 345                 350
   Gly Leu Ala Leu Ala Arg Val Arg Ala Pro Ser Asp Thr Thr Asn Gly
           355                 360                 365
   Thr Ser Ser Ser Lys Gly Ser Ser His Asn Lys Gly Gln Arg Ala Ser
       370                 375                 380
   Ser Ser Thr Tyr His Arg Gln Arg Arg His Ser Asp Phe Cys Gly Pro
   385                 390                 395                 400
   Ser Pro Ala Pro Leu His Pro Lys Arg Thr Pro Thr Ser Thr Gly Asp
                   405                 410                 415
   Thr Glu Leu Lys Glu Glu Arg Met Pro Gly Arg Lys Ala Ser Cys Ser
               420                 425                 430
   Ala Val Gly Ser Gly Ser Arg Gly Leu Pro Pro Ser Ser Pro Met Val
           435                 440                 445
   Ser Ser Ala His Asn Pro Asn Lys Ala Glu Ile Pro Glu Arg Arg Lys
       450                 455                 460
   Asp Ser Thr Ser Thr Pro Asn Asn Leu Pro Pro Ser Met Met Thr Arg
   465                 470                 475                 480
   Arg Asn Thr Tyr Val Cys Thr Glu Arg Pro Gly Ser Glu Arg Pro Ser
                   485                 490                 495
   Leu Leu Pro Asn Gly Lys Glu Asn Ser Ser Gly Thr Ser Arg Val Pro
               500                 505                 510
   Pro Ala Ser Pro Ser Ser His Ser Leu Ala Pro Pro Ser Gly Glu Arg
           515                 520                 525
   Ser Arg Leu Ala Arg Gly Ser Thr Ile Arg Ser Thr Phe His Gly Gly
       530                 535                 540
   Gln Val Arg Asp Arg Arg Ala Gly Ser Gly Ser Gly Gly Gly Val Gln
   545                 550                 555                 560
   Asn Gly Pro Pro Ala Ser Pro Thr Leu Ala His Glu Ala Ala Pro Leu
                   565                 570                 575
   Pro Ser Gly Arg Pro Arg Pro Thr Thr Asn Leu Phe Thr Lys Leu Thr
               580                 585                 590
   Ser Lys Leu Thr Arg Arg Val Thr Asp Glu Pro Glu Arg Ile Gly Gly
           595                 600                 605
   Pro Glu Val Thr Ser Cys His Leu Pro Trp Asp Lys Thr Glu Thr Ala
       610                 615                 620
   Pro Arg Leu Leu Arg Phe Pro Trp Ser Val Lys Leu Thr Ser Ser Arg
   625                 630                 635                 640
   Pro Pro Glu Ala Leu Met Ala Ala Met Arg Gln Ala Thr Ala Ala Ala
                   645                 650                 655
   Arg Cys Arg Cys Arg Gln Pro Gln Pro Phe Leu Leu Ala Cys Leu His
               660                 665                 670
   Gly Gly Ala Gly Gly Pro Glu Pro Leu Ser His Phe Glu Val Glu Val
           675                 680                 685
   Cys Gln Leu Pro Arg Pro Gly Leu Arg Gly Val Leu Phe Arg Arg Val
       690                 695                 700
   Ala Gly Thr Ala Leu Ala Phe Arg Thr Leu Val Thr Arg Ile Ser Asn
   705                 710                 715                 720
   Asp Leu Glu Leu
<210> 6
   <211> 660
   <212> PRT
   <213> Mus musculus
<400> 6
   Lys Gly Pro Ser Trp Ser Ser Arg Ser Leu Gly Ala Arg Cys Arg Asn
    1               5                  10                  15
   Ser Ile Ala Ser Cys Pro Glu Glu Gln Pro His Val Gly Asn Tyr Arg
               20                  25                  30
   Leu Leu Arg Thr Ile Gly Lys Gly Asn Phe Ala Lys Val Lys Leu Ala
           35                  40                  45
   Arg His Ile Leu Thr Gly Arg Glu Val Ala Ile Lys Ile Ile Asp Lys
       50                  55                  60
   Thr Gln Leu Asn Pro Ser Ser Leu Gln Lys Leu Phe Arg Glu Val Arg
   65                  70                  75                  80
   Ile Met Lys Gly Leu Asn His Pro Asn Ile Val Lys Leu Phe Glu Val
                   85                  90                  95
   Ile Glu Thr Glu Lys Thr Leu Tyr Leu Val Met Glu Tyr Ala Ser Ala
               100                 105                 110
   Gly Glu Val Phe Asp Tyr Leu Val Ser His Gly Arg Met Lys Glu Lys
           115                 120                 125
   Glu Ala Arg Ala Lys Leu Arg Gln Ile Val Ser Ala Val His Tyr Cys
       130                 135                 140
   His Gln Lys Asn Ile Val His Arg Asp Leu Lys Ala Glu Asn Leu Leu
   145                 150                 155                 160
   Leu Asp Ala Glu Ala Asn Ile Lys Ile Ala Asp Phe Gly Phe Ser Asn
                   165                 170                 175
   Glu Phe Thr Leu Gly Ser Lys Leu Asp Thr Phe Cys Gly Ser Pro Pro
               180                 185                 190
   Tyr Ala Ala Pro Glu Leu Phe Gln Gly Lys Lys Tyr Asp Gly Pro Glu
           195                 200                 205
   Val Asp Ile Trp Ser Leu Gly Val Ile Leu Tyr Thr Leu Val Ser Gly
       210                 215                 220
   Ser Leu Pro Phe Asp Gly His Asn Leu Lys Glu Leu Arg Glu Arg Ile
   225                 230                 235                 240
   Leu Arg Gly Lys Tyr Arg Val Pro Phe Tyr Met Ser Thr Asp Cys Glu
                   245                 250                 255
   Ser Ile Leu Arg Arg Phe Leu Val Leu Asn Pro Ala Lys Arg Cys Thr
               260                 265                 270
   Leu Glu Gln Ile Met Lys Asp Lys Trp Ile Asn Ile Gly Tyr Glu Gly
           275                 280                 285
   Glu Glu Leu Lys Pro Tyr Thr Glu Pro Glu Glu Asp Phe Gly Asp Thr
   290 295 300
   Lys Arg Ile Glu Val Met Val Gly Met Gly Tyr Thr Arg Glu Glu Ile
   305 310 315 320
   Lys Glu Ala Leu Thr Asn Gln Lys Tyr Asn Glu Val Thr Ala Thr Tyr
   325 330 335
   Leu Leu Leu Gly Arg Lys Thr Asp Glu Gly Gly Asp Arg Gly Ala Pro
   340 345 350
   Gly Leu Ala Leu Ala Arg Val Arg Ala Pro Ser Asp Thr Thr Asn Gly
   355 360 365
   Thr Ser Ser Ser Lys Gly Ser Ser His Asn Lys Gly Gln Arg Ala Ser
   370 375 380
   Ser Ser Thr Tyr His Arg Gln Arg Arg His Ser Asp Phe Cys Gly Pro
   385 390 395 400
   Ser Pro Ala Pro Leu His Pro Lys Arg Ser Pro Thr Ser Thr Gly Asp
   405 410 415
   Thr Glu Leu Lys Glu Glu Arg Met Pro Gly Arg Lys Ala Ser Cys Ser
   420 425 430
   Ala Val Gly Ser Gly Ser Arg Gly Leu Pro Pro Ser Ser Pro Met Val
   435 440 445
   Ser Ser Ala His Asn Pro Asn Lys Ala Glu Ile Pro Glu Arg Arg Lys
   450 455 460
   Asp Ser Thr Ser Thr Pro Asn Asn Leu Pro Pro Ser Met Met Thr Arg
   465 470 475 480
   Arg Asn Thr Tyr Val Cys Thr Glu Arg Pro Gly Ser Glu Arg Pro Ser
   485 490 495
   Leu Leu Pro Asn Gly Lys Glu Asn Ser Ser Gly Thr Ser Arg Val Pro
   500 505 510
   Pro Ala Ser Pro Ser Ser His Ser Leu Ala Pro Pro Ser Gly Glu Arg
   515 520 525
   Ser Arg Leu Ala Arg Gly Ser Thr Ile Arg Ser Thr Phe His Gly Gly
   530 535 540
   Gln Val Arg Asp Arg Arg Ala Gly Ser Gly Ser Gly Gly Gly Val Gln
   545 550 555 560
   Asn Gly Pro Pro Ala Ser Pro Thr Leu Ala His Glu Ala Ala Pro Leu
   565 570 575
   Pro Ser Gly Arg Pro Arg Pro Thr Thr Asn Leu Phe Thr Lys Leu Thr
   580 585 590
   Ser Lys Leu Thr Arg Arg Val Thr Leu Asp Pro Ser Lys Arg Gln Asn
   595 600 605
   Ser Asn Arg Cys Val Ser Gly Ala Ser Leu Pro Gln Gly Ser Lys Ile
   610 615 620
   Arg Ser Gln Thr Asn Leu Arg Glu Ser Gly Asp Leu Arg Ser Gln Val
   625 630 635 640
   Ala Ile Tyr Leu Gly Ile Lys Arg Lys Pro Pro Pro Gly Cys Ser Asp
   645 650 655
   Ser Pro Gly Val
   660
<210> 7
   <211> 752
   <212> PRT
   <213> Homo sapiens
<400> 7
   Met Ser Ser Arg Thr Val Leu Ala Pro Gly Asn Asp Arg Asn Ser Asp
   1 5 10 15
   Thr His Gly Thr Leu Gly Ser Gly Arg Ser Ser Asp Lys Gly Pro Ser
   20 25 30
   Trp Ser Ser Arg Ser Leu Gly Ala Arg Cys Arg Asn Ser Ile Ala Ser
   35 40 45
   Cys Pro Glu Glu Gln Pro His Val Gly Asn Tyr Arg Leu Leu Arg Thr
   50 55 60
   Ile Gly Lys Gly Asn Phe Ala Lys Val Lys Leu Ala Arg His Ile Leu
   65 70 75 80
   Thr Gly Arg Glu Val Ala Ile Lys Ile Ile Asp Lys Thr Gln Leu Asn
   85 90 95
   Pro Ser Ser Leu Gln Lys Leu Phe Arg Glu Val Arg Ile Met Lys Gly
   100 105 110
   Leu Asn His Pro Asn Ile Val Lys Leu Phe Glu Val Ile Glu Thr Glu
   115 120 125
   Lys Thr Leu Tyr Leu Val Met Glu Tyr Ala Ser Ala Gly Glu Val Phe
   130 135 140
   Asp Tyr Leu Val Ser His Gly Arg Met Lys Glu Lys Glu Ala Arg Ala
   145 150 155 160
   Lys Phe Arg Gln Ile Val Ser Ala Val His Tyr Cys His Gln Lys Asn
   165 170 175
   Ile Val His Arg Asp Leu Lys Ala Glu Asn Leu Leu Leu Asp Ala Glu
   180 185 190
   Ala Asn Ile Lys Ile Ala Asp Phe Gly Phe Ser Asn Glu Phe Thr Leu
   195 200 205
   Gly Ser Lys Leu Asp Thr Phe Cys Gly Ser Pro Pro Tyr Ala Ala Pro
   210 215 220
   Glu Leu Phe Gln Gly Lys Lys Tyr Asp Gly Pro Glu Val Asp Ile Trp
   225 230 235 240
   Ser Leu Gly Val Ile Leu Tyr Thr Leu Val Ser Gly Ser Leu Pro Phe
   245 250 255
   Asp Gly His Asn Leu Lys Glu Leu Arg Glu Arg Val Leu Arg Gly Lys
   260 265 270
   Tyr Arg Val Pro Phe Tyr Met Ser Thr Asp Cys Glu Ser Ile Leu Arg
   275 280 285
   Arg Phe Leu Val Leu Asn Pro Ala Lys Arg Cys Thr Leu Glu Gln Ile
   290 295 300
   Met Lys Asp Lys Trp Ile Asn Ile Gly Tyr Glu Gly Glu Glu Leu Lys
   305 310 315 320
   Pro Tyr Thr Glu Pro Glu Glu Asp Phe Gly Asp Thr Lys Arg Ile Glu
   325 330 335
   Val Met Val Gly Met Gly Tyr Thr Arg Glu Glu Ile Lys Glu Ser Leu
   340 345 350
   Thr Ser Gln Lys Tyr Asn Glu Val Thr Ala Thr Tyr Leu Leu Leu Gly
   355 360 365
   Arg Lys Thr Glu Glu Gly Gly Asp Arg Gly Ala Pro Gly Leu Ala Leu
   370 375 380
   Ala Arg Val Arg Ala Pro Ser Asp Thr Thr Asn Gly Thr Ser Ser Ser
   385 390 395 400
   Lys Gly Thr Ser His Ser Lys Gly Gln Arg Ser Ser Ser Ser Thr Tyr
   405 410 415
   His Arg Gln Arg Arg His Ser Asp Phe Cys Gly Pro Ser Pro Ala Pro
   420 425 430
   Leu His Pro Lys Arg Ser Pro Thr Ser Thr Gly Glu Ala Glu Leu Lys
   435 440 445
   Glu Glu Arg Leu Pro Gly Arg Lys Ala Ser Cys Ser Thr Ala Gly Ser
   450 455 460
   Gly Ser Arg Gly Leu Pro Pro Ser Ser Pro Met Val Ser Ser Ala His
   465 470 475 480
   Asn Pro Asn Lys Ala Glu Ile Pro Glu Arg Arg Lys Asp Ser Thr Ser
   485 490 495
   Thr Pro Asn Asn Leu Pro Pro Ser Met Met Thr Arg Arg Asn Thr Tyr
   500 505 510
   Val Cys Thr Glu Arg Pro Gly Ala Glu Arg Pro Ser Leu Leu Pro Asn
   515 520 525
   Gly Lys Glu Asn Ser Ser Gly Thr Pro Arg Val Pro Pro Ala Ser Pro
   530 535 540
   Ser Ser His Ser Leu Ala Pro Pro Ser Gly Glu Arg Ser Arg Leu Ala
   545 550 555 560
   Arg Gly Ser Thr Ile Arg Ser Thr Phe His Gly Gly Gln Val Arg Asp
   565 570 575
   Arg Arg Ala Gly Gly Gly Gly Gly Gly Gly Val Gln Asn Gly Pro Pro
   580 585 590
   Ala Ser Pro Thr Leu Ala His Glu Ala Ala Pro Leu Pro Ala Gly Arg
   595 600 605
   Pro Arg Pro Thr Thr Asn Leu Phe Thr Lys Leu Thr Ser Lys Leu Thr
   610 615 620
   Arg Arg Val Ala Asp Glu Pro Glu Arg Ile Gly Gly Pro Glu Val Thr
   625 630 635 640
   Ser Cys His Leu Pro Trp Asp Gln Thr Glu Thr Ala Pro Arg Leu Leu
   645 650 655
   Arg Phe Pro Trp Ser Val Lys Leu Thr Ser Ser Arg Pro Pro Glu Ala
   660 665 670
   Leu Met Ala Ala Leu Arg Gln Ala Thr Ala Ala Ala Arg Cys Arg Cys
   675 680 685
   Arg Gln Pro Gln Pro Phe Leu Leu Ala Cys Leu His Gly Gly Ala Gly
   690 695 700
   Gly Pro Glu Pro Leu Ser His Phe Glu Val Glu Val Cys Gln Leu Pro
   705 710 715 720
   Arg Pro Gly Leu Arg Gly Val Leu Phe Arg Arg Val Ala Gly Thr Ala
   725 730 735
   Leu Ala Phe Arg Thr Leu Val Thr Arg Ile Ser Asn Asp Leu Glu Leu
   740 745 750
<210> 8
   <211> 688
   <212> PRT
   <213> Homo sapiens
<400> 8
   Met Ser Ser Arg Thr Val Leu Ala Pro Gly Asn Asp Arg Asn Ser Asp
   1               5                   10                  15
   Thr His Gly Thr Leu Gly Ser Gly Arg Ser Ser Asp Lys Gly Pro Ser
               20                  25                  30
   Trp Ser Ser Arg Ser Leu Gly Ala Arg Cys Arg Asn Ser Ile Ala Ser
           35                  40                  45
   Cys Pro Glu Glu Gln Pro His Val Gly Asn Tyr Arg Leu Leu Arg Thr
       50                  55                  60
   Ile Gly Lys Gly Asn Phe Ala Lys Val Lys Leu Ala Arg His Ile Leu
   65                  70                  75                  80
   Thr Gly Arg Glu Val Ala Ile Lys Ile Ile Asp Lys Thr Gln Leu Asn
                   85                  90                  95
   Pro Ser Ser Leu Gln Lys Leu Phe Arg Glu Val Arg Ile Met Lys Gly
               100                 105                 110
   Leu Asn His Pro Asn Ile Val Lys Leu Phe Glu Val Ile Glu Thr Glu
           115                 120                 125
   Lys Thr Leu Tyr Leu Val Met Glu Tyr Ala Ser Ala Gly Glu Val Phe
       130                 135                 140
   Asp Tyr Leu Val Ser His Gly Arg Met Lys Glu Lys Glu Ala Arg Ala
   145                 150                 155                 160
   Lys Phe Arg Gln Ile Val Ser Ala Val His Tyr Cys His Gln Lys Asn
                   165                 170                 175
   Ile Val His Arg Asp Leu Lys Ala Glu Asn Leu Leu Leu Asp Ala Glu
               180                 185                 190
   Ala Asn Ile Lys Ile Ala Asp Phe Gly Phe Ser Asn Glu Phe Thr Leu
           195                 200                 205
   Gly Ser Lys Leu Asp Thr Phe Cys Gly Ser Pro Pro Tyr Ala Ala Pro
       210                 215                 220
   Glu Leu Phe Gln Gly Lys Lys Tyr Asp Gly Pro Glu Val Asp Ile Trp
   225                 230                 235                 240
   Ser Leu Gly Val Ile Leu Tyr Thr Leu Val Ser Gly Ser Leu Pro Phe
                   245                 250                 255
   Asp Gly His Asn Leu Lys Glu Leu Arg Glu Arg Val Leu Arg Gly Lys
               260                 265                 270
   Tyr Arg Val Pro Phe Tyr Met Ser Thr Asp Cys Glu Ser Ile Leu Arg
           275                 280                 285
   Arg Phe Leu Val Leu Asn Pro Ala Lys Arg Cys Thr Leu Glu Gln Ile
       290                 295                 300
   Met Lys Asp Lys Trp Ile Asn Ile Gly Tyr Glu Gly Glu Glu Leu Lys
   305                 310                 315                 320
   Pro Tyr Thr Glu Pro Glu Glu Asp Phe Gly Asp Thr Lys Arg Ile Glu
                   325                 330                 335
   Val Met Val Gly Met Gly Tyr Thr Arg Glu Glu Ile Lys Glu Ser Leu
               340                 345                 350
   Thr Ser Gln Lys Tyr Asn Glu Val Thr Ala Thr Tyr Leu Leu Leu Gly
           355                 360                 365
   Arg Lys Thr Glu Glu Gly Gly Asp Arg Gly Ala Pro Gly Leu Ala Leu
       370                 375                 380
   Ala Arg Val Arg Ala Pro Ser Asp Thr Thr Asn Gly Thr Ser Ser Ser
   385                 390                 395                 400
   Lys Gly Thr Ser His Ser Lys Gly Gln Arg Ser Ser Ser Ser Thr Tyr
                   405                 410                 415
   His Arg Gln Arg Arg His Ser Asp Phe Cys Gly Pro Ser Pro Ala Pro
                    420                     425                     430
   Leu His Pro Lys Arg Ser Pro Thr Ser Thr Gly Glu Ala Glu Leu Lys
           435                 440                     445
   Glu Glu Arg Leu Pro Gly Arg Lys Ala Ser Cys Ser Thr Ala Gly Ser
       450                     455                     460
   Gly Ser Arg Gly Leu Pro Pro Ser Ser Pro Met Val Ser Ser Ala His
   465                     470                     475                     480
   Asn Pro Asn Lys Ala Glu Ile Pro Glu Arg Arg Lys Asp Ser Thr Ser
                   485                     490                     495
   Thr Pro Asn Asn Leu Pro Pro Ser Met Met Thr Arg Arg Asn Thr Tyr
               500                     505                     510
   Val Cys Thr Glu Arg Pro Gly Ala Glu Arg Pro Ser Leu Leu Pro Asn
           515                     520                     525
   Gly Lys Glu Asn Ser Ser Gly Thr Pro Arg Val Pro Pro Ala Ser Pro
       530                     535                     540
   Ser Ser His Ser Leu Ala Pro Pro Ser Gly Glu Arg Ser Arg Leu Ala
   545                     550                     555                     560
   Arg Gly Ser Thr Ile Arg Ser Thr Phe His Gly Gly Gln Val Arg Asp
                   565                     570                     575
   Arg Arg Ala Gly Gly Gly Gly Gly Gly Gly Val Gln Asn Gly Pro Pro
               580                     585                     590
   Ala Ser Pro Thr Leu Ala His Glu Ala Ala Pro Leu Pro Ala Gly Arg
           595                     600                     605
   Pro Arg Pro Thr Thr Asn Leu Phe Thr Lys Leu Thr Ser Lys Leu Thr
       610                     615                     620
   Arg Arg Val Thr Leu Asp Pro Ser Lys Arg Gln Asn Ser Asn Arg Cys
   625                     630                     635                     640
   Val Ser Gly Ala Ser Leu Pro Gln Gly Ser Lys Ile Arg Ser Gln Thr
                   645                     650                     655
   Asn Leu Arg Glu Ser Gly Asp Leu Arg Ser Gln Val Ala Ile Tyr Leu
               660                     665                     670
   Gly Ile Lys Arg Lys Pro Pro Pro Gly Cys Ser Asp Ser Pro Gly Val
           675                     680                     685

## Beschreibung

Die vorliegende Erfindung betrifft eine Serin-Threonin-Protein-Kinase, Nukleinsäuren, die diese Kinase kodieren und deren Verwendung zur Diagnose und Therapie neuronaler und neoplastischer Erkrankungen.

Apoplexie, auch Schlaganfall, Gehirnschlag, apoplektischer Insult genannt, ist bei ca. 15% aller Todesfälle beteiligt, wobei Männer und Frauen gleichermaßen betroffen sind. Die Symptome umfassen Bewußtseinsstörungen bis zum Koma, häufig auch spastische Hemiplegie, verschiedenste zentrale motorische, sensible und sensorische Ausfallerscheinungen sowie fokale oder generalisierte Krampfanfälle. In jedem Fall einer Apoplexie handelt es sich um eine mit Sauerstoffmangel einhergehende Kreislaufstörung im Bereich einer umschriebenen Hirnregion. Als Auslöser kommen zwei grundsätzliche Mechanismen in Betracht. Erstens, eine Massenblutung, Enzephalorrhagie, die in 15% der Fälle beteiligt ist, wobei diese nach Gefäßruptur meist der strio-lentikulären Arterien auftritt, wodurch begrenzte Hirnregionen zerstört werden. Dieser Mechanismus hat eine hohe Letalität zur Folge. Als Grundleiden kommen insbesondere Hypertonie, Arteriosklerose, Intrakranielles Aneurysma und in selteneren Fällen eine Verbrauchskoagulopathie in Frage. Als zweiter Mechanismus ist ein Hirninfarkt, Enzephalomalazie, beteiligt, wobei dies in 85% der Fälle als Ursache angesehen wird. Dabei kommt es in der Regel zur Ausbildung einer Nekrose. Ursache hierfür sind u.a. eine arterielle Thrombose, Thromboembolie oder funktionelle Ischämie bei offenem Gefäßlumen, z.B. in Folge eines Blutdruckabfalls. Der Hirninfarkt "ischämische Nekrose" ist zu etwa 70 - 80% die Ursache der Apoplexie. Häufig stellt Arteriosklerose das zugrundeliegende ursächliche Leiden dar. Die seltene, sich langsam entwickelnde Symptomatik einer Enzephalomalazie wird als "progressive stroke" bezeichnet. Als Vorboten eines Himinfarkts sind passager auftretende neurologische Ausfallssymptome ohne Ausbildung von Gewebsschäden zu bewerten ("transitorische ischämische Attacken"). Als Ursache wird eine vorübergehende stenotisch bedingte oder durch Mikroembolien verursachte begrenzte Durchblutungsstörung angenommen. Die Diagnose umfasst neben einer allgemeinen und neurologischen Untersuchung auch kraniale Computer-Tomographie, zerebrovaskuläre Doppler-Ultraschalluntersuchung, Lumbalpunktion, dynamische Hirnszinthigraphie, EEG sowie Kemspinresonanz-Tomographie.

Die molekularen Grundlagen der Ischämie und der damit verbundenen Folgeerscheinungen sind bis zum heutigen Tage nahezu unbekannt. Es ist jedoch davon auszugehen, dass eine komplexe Folge an biochemischen Vorgängen bis zum Auftreten der Apoplexie erforderlich ist.

Der vorliegenden Erfindung lag das technische Problem zugrunde, Gene zu identifizieren, die an der Entstehung der Apoplexie in Folge lokalen Sauerstoffmangels beteiligt sind, und somit neue Prophylaxe- und Therapiemöglichkeiten der Apoplexie zu eröffnen. Ferner lag der vorliegenden Erfindung das technische Problem zugrunde, Proteine zu identifizieren, die an der Entstehung der Apoplexie beteiligt sind.

Die genannten technischen Probleme werden gelöst durch eine Nukleinsäure, die für eine Serin-Threonin-Protein-Kinase kodiert, wobei die Nukleinsäure ausgewählt wird aus:
a) Nukleinsäure, mit einer der Sequenzen nach SEQ ID Nr. 1 - 4 und Nukleinsäure, die für ein Protein mit einer Sequenz gemäß einer der SEQ ID Nr. 5 - 8 kodiert;
b) Nukleinsäure, die für ein Protein kodiert, das mindestens 70% Identität zu einem Protein gemäß einer der SEQ ID Nr. 5 - 8 aufweist, und dessen Expression durch fokale cerebrale Ischämie und Reperfusion induziert werden kann.

Weiterhin werden folgende Nukleinsäuren beschrieben:
i) Nukleinsäure, die mit einer Nukleinsäure nach a) hybridisiert;
ii) Nukleinsäure, die unter Berücksichtigung der Degeneration des genetischen Codes mit einer Nukleinsäure nach a) hybridisieren würde;
iii) durch Substitution, Addition, Inversion und/oder Deletion einer oder mehrerer Basen erhaltener Derivate einer Nukleinsäure nach a) - c); und komplementäre Nukleinsäure zu einer Nukleinsäure nach a) - d).

Beispielsweise werden in Derivaten der Proteine gemäß SEQ ID Nr. 5 - 8 Argininreste gegen Lysinreste, Valinreste gegen Isoleucinreste und Asparaginsäurereste gegen Glutaminsäurereste ausgetauscht. Dabei sind die physikochemischen Eigenchaften der ausgetauschten und austauschenden Aminosäure sehr ähnlich (z.B. die Raumerfüllung, Basizität, Hydrophobizität). Es können aber auch eine oder mehrere Aminosäuren in ihrer Reihenfolge vertauscht, hinzugefügt oder entfernt werden, oder es können mehrere dieser Maßnahmen miteinander kombiniert werden. Die solchermaßen gegenüber der SEQ ID Nr. 5-8 veränderten Proteine besitzen, bevorzugt wenigstens 70% und besonders bevorzugt wenigstens 90% Sequenzidentität zu den Sequenzen SEQ ID Nr. 5-8, berechnet nach dem Algorithmus von Altschul et al., J. Mol. Biol., 215, 403-410, 1990. Das isolierte Protein und seine funktionellen Varianten lassen sich vorteilhafterweise aus dem Gehirn von Mammalia wie Homo sapiens, Rattus norvegicus oder Mus musculus isolieren. Auch Homologe aus anderen Mammalia sind unter funktionellen Varianten zu verstehen.

Die erfindungsgemäßen Nukleinsäuren gemäß SEQ ID Nr. 1 - 4 stellen Nukleinsäuren dar, wie sie aus Maus (SEQ ID Nr. 1 und 2) bzw. Mensch (SEQ ID Nr. 3 und 4) isoliert werden. Dabei sind die Nukleinsäuren gemäß SEQ ID Nr. 2 und SEQ ID Nr. 4 längere Spleissvarianten der SEQ ID Nr. 1 bzw. SEQ ID Nr. 3. Als erfindungsgemäß werden auch solche Nukleinsäuren angesehen, die für ein Protein kodieren, das vorzugsweise mindestens 70%, ganz besonders bevorzugt mindestens 90% Identität zu einem der Proteine wie durch SEQ ID Nr. 1 - 4 kodiert aufweist.

Erfindungsgemäße "Derivate" der oben genannten Nukleinsäuren, z.B. Allelvarianten, unterscheiden sich von den genannten Nukleinsäuren gemäß SEQ ID Nr. 1 - 4 durch Substitution, Addition, Inversion und/oder Deletion einer oder mehrerer Basen, wobei jedoch die Kinase-Aktivität beibehalten wird. Derivate, wie Homologe oder sequenzverwandte Nukleinsäuresequenzen können aus allen Säugerspezies, einschließlich Mensch, nach gängigen Verfahren durch Hybridisierung mit einer der erfindungsgemäßen Nukleinsäuresequenzen oder Fragmenten davon isoliert werden.

Unter "funktionellen Äquivalenten" sind auch Homologe der Nukleinsäure gemäß SEQ ID Nr. 1 - 4, beispielsweise Homologe aus anderen Mammalia, verkürzte Sequenzen, Einzelstrang - DNA oder RNA oder PNA der codierenden und nicht codierenden Nukleinsäuresequenz zu verstehen. Solche funktionellen Äquivalente lassen sich ausgehend von den Nukleinsäuren der SEQ ID Nr. 1 - 4, beispielsweise mit üblichen Hybridisierungsverfahren oder der PCR-Technik, aus anderen Vertebraten, wie Mammalia, isolieren. Zur Hybridisierung werden vorteilhaft Oligonukleotide konservierter Bereiche, die vom Fachmann in bekannter Weise ermittelt werden können, verwendet. Es können aber auch längere Fragmente der erfindungsgemäßen Nukleinsäuren oder die vollständige Sequenz für die Hybridisierung verwendet werden. Je nach der verwendeten Nukleinsäure - Oligonukleotid, längeres Fragment oder vollständige Sequenz - oder je nachdem, welche Nukleinsäureart - DNA oder RNA - für die Hybridisierung verwendet werden, variieren die Standardbedingungen zur Hybridisierung. So liegen beispielsweise Schmelztemperaturen für DNA: DNA-Hybride ca. 10° niedriger als die von DNA-RNA-Hybriden gleicher Länge.

Unter "Standardbedingungen" sind beispielsweise je nach Nukleinsäuretemperaturen zwischen 42 und 58°C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Unter dem Begriff "Derivate" der erfindungsgemäßen Sequenzen nach SEQ ID Nr. 1 - 4 sind auch Promotor-Varianten zu verstehen, die den kodierenden Bereichen der erfindungsgemäßen Sequenzen vorgeschaltet sind; diese können durch ein oder mehrere Nukleotidaustausche durch Insertion, Addition und/oder Deletion verändert sein, ohne dass die Promotor-Eigenschaften, insbesondere die Promotorstärke und Induzierbarkeit beeinträchtigt wird. Der Begriff Derivat umfasst jedoch auch solche Promotor-Varianten, die ausgehend von den erfindungsgemäßen Sequenzen durch Nukleotidaustausch(e) in ihrer Aktivität verstärkt werden.

"Neoplastische Erkrankungen" sind solche, die mit abnormen Wachstumsverhalten von Zellen, Wegfall interzellulärer Hemmmechanismen etc. zu tun haben. Dazu zählen z.B. Carcinome als abnorme Proliferationen endodermaler Zellen, lymphoneoplastische Erkrankungen, Melanome etc.

Eine bevorzugte Nukleinsäure kodiert für ein Protein mit einer Sequenz gemäß einer SEQ ID Nr. 5 - 8 oder eine Protein, das mindestens 70% Identität zu einer der genannten Sequenzen aufweist.

Bei einer weiteren bevorzugten Ausführungsform ist die Nukleinsäure zu mindestens 70% identisch zu den kodierenden Abschnitten einer der Sequenzen nach einer der SEQ ID Nr. 1 - 4.

Bei einer weiteren bevorzugten Ausführungsform kodiert die Nukleinsäure eine Proteinsequenz nach einem der SEQ ID Nr. 5 - 8, wobei eine Nukleinsäure, die für die SEQ ID Nr. 7 kodiert besonders bevorzugt.

Vorzugsweise ist die erfindungsgemäße Nukleinsäure eine DNA, es kommen jedoch auch RNA oder PNA in Betracht.

Als erfindungsgemäße Nukleinsäure sind insbesondere solche bevorzugt, die geeignet sind, in Antisinnorientierung zu einem Promotor nach Einbringen in eine Wirtszelle die Expression einer Serin-Threonin-Kinase zu hemmen.

Erfindungsgemäße Konstrukte enthalten die erfindungsgemäße Nukleinsäuresequenz in Kombination mit weiteren Sequenzen, mit denen sie üblicherweise im Genom einer Wirtszelle nicht assoziiert sind. Solche "Fremdsequenzen" sind vorzugsweise genetische Regulationselemente, die Transkriptions- und Translationssignale (auch als "Expression regulierende Elemente" bezeichnet oder von Vektoren abgeleitete Sequenzen). Mit diesen Elementen sind die erfindungsgemäßen Sequenzen funktionell verknüpft.

Diese Verknüpfung kann je nach gewünschter Anwendung zu einer Erhöhung oder Erniedrigung der Genexpression führen. Mit den solchermaßen hergestellten rekombinanten Nukleinsäurekonstrukten können Wirtsorganismen transformiert werden. Zusätzlich zu diesen Regulationssequenzen kann die natürliche Regulation dieser Sequenzen vor den eigentlichen Strukturgenen noch vorhanden sein und gegebenenfalls genetisch verändert worden sein, so daß die natürliche Regulation ausgeschaltet und die Expression der Gene erhöht wurde. Das Genkonstrukt kann aber auch einfacher aufgebaut sein, das heißt es werden keine zusätzlichen Regulationssignale vor die Sequenzen inseriert und der natürliche Promotor mit seiner Regulation wird nicht entfernt. Stattdessen kann die natürliche Regulationssequenz so mutiert werden, daß keine Regulation mehr erfolgt und die Genexpression gesteigert wird. Auch am 3'-Ende der erfindungsgemäßen Nukleinsäure-Sequenzen können zusätzliche vorteilhafte regulatorische Elemente inseriert werden. Die Nukleinsäuresequenzen nach SEQ ID Nr. 1 - 4 und/oder für die entsprechenden Proteine kodierende Sequenzen nach SEQ ID Nr. 5 - 8 können in einer oder mehreren Kopien im Genkonstrukt enthalten sein, oder auf getrennten Genkonstrukten lokalisiert sein. Vorteilhafte Regulationssequenzen sind beispielsweise in Promotoren wie cos-, tac-, trp-, tet-, trp-tet-, Ipp-, lac-, Ipp-lac-, laclq-, T7-, T5-, T3-, gal-, trc-, ara-, SP6-, I-PR- oder im I-PL-Promotor enthalten, die vorzugsweise in gramnegativen Bakterien Anwendung finden. Weitere vorteilhafte Regulationssequenzen sind beispielsweise in den gram-positiven Promotoren wie amy und SPO2, in den Hefepromotoren wie ADC1, MFa , AC, P-60, CYC1, GAPDH oder in Mammaliapromotoren wie CaM-Kinase 11, CMV, Nestin, L7, BDNF, NF, MBP, NSE, beta-Globin, GFAP, GAP43, Tyrosin Hydroxylase, Kainat-Rezeptor-Untereinheit 1, Glutamat-Rezeptor-Untereinheit B enthalten. Prinzipiell können alle natürlichen Promotoren mit ihren Regulationssequenzen wie die oben genannten verwendet werden. Darüberhinaus können auch synthetische Promotoren vorteilhaft verwendet werden. Diese regulatorischen Sequenzen sollen die gezielte Expression der Nukleinsäuresequenzen und der Proteinexpression ermöglichen. Dies kann beispielsweise je nach Wirtsorganismus bedeuten, daß das Gen erst nach Induktion exprimiert oder überexprimiert wird, oder daß es sofort exprimiert und/oder überexprimiert wird. Die regulatorischen Sequenzen bzw. Faktoren können dabei vorzugsweise die Expression positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird. Unter "Enhancer" sind beispielsweise DNA-Sequenzen zu verstehen, die über eine verbesserte Wechselwirkung zwischen RNA-Polymerase und DNA eine erhöhte Expression bewirken. Als weitere Regulationssequenzen seien beispielhaft die "locus control regions", "silencer" oder jeweilige Teilsequenzen davon genannt. Diese Sequenzen können vorteilhaft für eine gewebespezifische Expression verwendet werden. Eine bevorzugte Ausführungsform ist die Verknüpfung der erfindungsgemäßen Nukleinsäuresequenz mit einem Promotor, wobei der Promotor 5' "upstream" zu liegen kommt. Weitere Regulationssignale wie 3'gelegene Terminatoren oder Polyadenylierungssignale oder Enhancer können funktionell in dem Nukleinsäurekonstrukt Anwendung finden. Unter dem Begriff sind auch komplette Vektorkonstrukte zu verstehen. Diese Vektorkonstrukte oder Vektoren werden zur Expression in einem geeigneten Wirtsorganismus verwendet. Vorteilhafterweise werden die erfindungsgemäßen Nukleinsäuren und/oder die Gene für die Ser-Thr-Proteinkinase in einen wirtsspezifischen Vektor inseriert, der eine optimale Expression der Gene im ausgesuchten Wirt ermöglicht. Vektoren sind dem Fachmann wohl bekannt und können beispielsweise aus dem Buch Cloning Vectors (Eds. Pouwels P. H. et al. Elsevier, Amsterdam-New York-Oxford, 1985, ISBN 0 444 904018) entnommen werden. Unter Vektoren sind außer Plasmiden auch alle anderen dem Fachmann bekannten Vektoren wie beispielsweise Phagen, Viren wie SV40, CMV, Baculovirus, A-denovirus, Sindbisvirus, Transposons, IS-Elemente, Phasmide, Phagemide, Cosmide, lineare oder zirkuläre DNA zu verstehen. Diese Vektoren können autonom im Wirtsorganismus repliziert oder chromosomal repliziert werden. Für die Integration in Mammalia wird vorteilhaft lineare DNA verwendet. Die Expression der erfindungsgemäßen Nukleinsäuresequenzen bzw. des rekombinanten Nukleinsäurekonstrukts kann vorteilhaft durch Erhöhen der Genkopienzahl und/oder durch Verstärkung regulatorischer Faktoren, die die Genexpression positiv beeinflussen, erhöht werden. So kann eine Verstärkung regulatorischer Elemente vorzugsweise auf der Transkriptionsebene erfolgen, indem stärkere Transkriptionssignale wie Promotoren und Enhancer verwendet werden. Daneben ist aber auch eine Verstärkung der Translation möglich, indem beispielsweise die Stabilität der mRNA verbessert, oder die Ableseeffizienz dieser mRNA an den Ribosomen erhöht wird. Zur Erhöhung der Genkopienzahl können die Nukleinsäuresequenzen oder homologe Gene, beispielsweise in ein Nukleinsäurefragment bzw. in einen Vektor eingebaut werden, der vorzugsweise die den jeweiligen Genen zugeordnete, regulatorische Gensequenzen oder analog wirkende Promotoraktivität enthält. lnsbesondere werden solche regulatorische Sequenzen verwendet, die die Genexpression verstärken. Die erfindungsgemäßen Nukleinsäuresequenzen können zusammen mit den für interagierende Proteine kodierenden Sequenzen in einen einzelnen Vektor kloniert werden und anschließend in dem gewünschten Organismus exprimiert werden. Alternativ kann auch jede der potentiell interagierenden Nukleinsäuresequenzen und die für m30 kodierenden Sequenzen in je einen einzelnen Vektor gebracht und diese getrennt in den jeweiligen Organismus über übliche Methoden wie Transformation, Transfektion, Transduktion, Elektroporation oder Partikel-Gun verbracht werden. Darüberhinaus kann das erfindungsgemäße Nukleinsäurekonstrukt oder die erfinungsgemäßen Nukleinsäuren auch in Form therapeutisch oder diagnostisch geeigneter Fragmente exprimiert werden. Zur Generierung des rekombinanten Proteins können Vektorsysteme oder Oligonukleotide verwendet werden, die die Nukleinsäuren oder das Nukleinsäurekonstrukt um bestimmte Nukleotidsequenzen verlängern und damit für veränderte Polypeptide kodieren, die einer einfacheren Reinigung dienen. Als solche "Tags" sind in der Literatur z. B. Hexa-Histidin -Anker bekannt oder Epitope, die als Antigene verschiedener Antikörper erkannt werden können (Studier et al., Meth. Enzymol., 185, 1990: 60 - 89 und Ausubel et al. (eds.) 1998, Current Protocols in Molecular Biology, John Wiley & Sons, New York).

Als Wirtszelle sind prinzipiell alle Zellen geeignet, die eine Expression der erfindungsgemäßen Nukleinsäuren, ihrer Allelvarianten, ihrer funktionellen Äquivalente oder Derivate oder des rekombinanten Nukieinsäurekonstrukts ermöglichen. Unter Wirtszellen sind beispielsweise Bakterien, Pilze, Hefen, pflanzliche oder tierische Zellen zu verstehen. Bevorzugte Wirtszellen/Organismen sind Bakterien wie Escherichia coli, Streptomyces, Bacillus oder Pseudomonas, eukaryotische Mikroorganismen wie Saccharomyces cerevisiae, Aspergillus, höhere eukaryotische Zellen aus Mensch oder Tier, beispielsweise COS-, Heia-, HEK293-, Sf9- oder CHO-Zellen. Die Kombination aus dem Wirtsorganismen und den zu den Organismen passenden Vektoren wie Plasmide, Viren oder Phagen wie beispielsweise Plasmide mit dem RNA-Polymerase/Promoter System, die Phagen 1, Mu oder andere temperänte Phagen oder Transposons und/oder weiteren vorteilhaften regulatorischen Sequenzen bilden ein Expressionssystem. Bevorzugt sind unter dem Begriff Expressionssysteme beispielsweise die Kombination aus Säugetierzellen wie CHO-Zellen und Vektoren wie pcDNA3neo-Vektor oder HEK293-Zellen und CMV-Vektor, die für Säugetierzellen geeignet sind, zu verstehen. Zellfreie, in vitro-Expressionssysteme kommen jedoch auch in Betracht.

Als erfindungsgemäße Wirte zur Expression der erfindungsgemäßen Nukleinsäuren kommt auch Säugergewebe, Säugerorgane bzw nicht-humane transgene Säuger in Betracht Die genannten Wirte unterscheiden sich vom Wildtyp vorzugsweise dadurch, dass sie im Vergleich zum Wildtyp eine veränderte Menge des erfindungsgemäßen Proteins enthalten oder aber eine neue Proteinvariante der erfindungsgemäßen Protein-Kinase. Umfasst sind jedoch auch Wirtsorganismen, bei denen die natürlich vorkommende Nukleinsäure, die für ein erfindungsgemäßes Protein kodiert entweder vollständig oder teilweise entfernt wurde oder so verändert wurde, dass sie transkriptionsinaktiv ist.

Vorzugsweise enthalten die genannten Organismen die erfindungsgemäße Nukleinsäure bzw. das erfindungsgemäße Konstrukt in einer Stelle des Genoms integriert, die nicht seiner natürlichen Position, wie im Wildtyp vorhanden, entspricht.

Als nicht-humane transgene Säuger kommen vorzugsweise Mäuse, Ratten, Schafe, Rinder oder Schweine in Betracht, wobei jedoch auch Nichtsäugerorganismen, wie Pflanzen, als Empfänger der erfindungsgemäßen Sequenzen in Betracht kommen. Bei den transgenen Organismen kann es sich auch um sogenannte Knock-Out Tiere handeln. Dabei können die transgenen Tiere eine funktionelle oder nicht funktionelle erfindungsgemäße Nukleinsäuresequenz oder ein funktionelles oder nicht funktionelles Nuldeinsäurekonstrukt enthalten. Eine weitere erfindungsgemäße Ausgestaltung der oben beschriebenen transgenen Tiere sind nicht-humane transgene Tiere, in deren Keimzellen oder der Gesamtheit oder einem Teil der somatischen Zellen; oder in dessen Keimzellen und der Gesamtheit oder einem Teil der somatischen Zellen die erfindungsgemäßen Nukleotidsequenz durch gentechnische Verfahren verändert oder durch Einfügen von DNA-Elementen unterbrochen wurden. Eine weitere Möglichkeit des Einsatzes der Nukleotidsequenz oder Teilen davon ist die Erzeugung transgener oder knock-out- oder konditioneller oder regionenspezfischer knock-out Tiere oder spezifischer Mutationen in gentechnisch veränderten Tieren (Ausubel et al. (eds.) 1998, Current Protocols in Molecular Biology, John Wiley & Sons, New York und Torres et al., (eds.) 1997, Laboratory protocols for conditional gene targeting, Oxford University Press, Oxford). Über transgene Überexpression oder genetische Mutation (Nullmutation oder spezifische Deletionen, Insertionen oder Veränderungen) durch homologe Rekombination in embryonalen Stammzellen kann man Tiermodelle erzeugen, die wertvolle weitere Informationen über die Pathogenese der Apoplexie liefern können. Solchermaßen hergestellte Tiermodelle können essentielle Testsysteme zur Evaluierung neuartiger Therapeutika darstellen.

Die erfindungsgemäßen Proteine sind erhältlich durch Expression einer der erfindungsgemäßen Nukleinsäuren in einem geeigneten Expressionssystem, wobei vorzugsweise intakte Zellen umfassende Expressionssysteme in Betracht kommen. Vorzugsweise ist das erfindungsgemäße Protein ein Protein ausgewählt aus einem Protein mit einer der Sequenzen nach SEQ ID Nr. 5 - 8 oder einem Protein, das zu den genannten Sequenzen mindestens 70%, besonders bevorzugt mindestens 90°% identisch ist. Dabei berechnet sich die "%-Identität" zu den Sequenzen nach SEQ ID Nr. 5 - 8 nach dem Algorithmus von Altschul et al., J. Mol. Biol, 250, 403 - 410, 1990. Das erfindungsgemäße Protein und funktionelle Varianten davon lässt sich jedoch auch aus dem Gehim von Mammalia wie Homo sapiens, Rattus norvegicus oder Mus musculus isolieren. Efindungsgemäße Proteine sind auch solche, die sich von einem Protein nach einer der SEQ ID Nr. 5 - 8 durch Aminosäureaustausch ableiten, wobei die Protein-Kinase-Aktivität im Wesentlichen unverändert bleibt. Beispielsweise können Aminosäuren in dem Ausgangsprotein gemäß SEQ ID Nr. 5 - 8 durch solche mit ähnlichen physikochemischen Eigenschaften (Raumerfüllung, Basizität, Hydrophobizität usw.) ersetzt werden. Beispielsweise können Argininreste gegen Lysinreste, Valinreste gegen Isoleucinreste oder Asparaginsäurereste gegen Glutaminsäurereste ausgetauscht werden. Es können aber auch eine oder mehrere Aminosäuren in ihrer Reihenfolge vertauscht, hinzugefügt oder entfernt werden, oder es können mehrere der genannten Maßnahmen miteinander kombiniert werden. Die Maßnahmen zum Verändern einer vorgegebenen Aminosäuresequenz zu einer gewünschten Sequenz sind dem Fachmann geläufig.

Das Herstellen der erfindungsgemäßen Antikörper, die mit einem erfindungsgemäßen Protein reagieren, ist dem Fachmann bekannt. Zu diesem Zweck kann er beispielsweise auf das Herstellen von polyklonalen Antiseren oder aber die Hybridom-Technologie zu Herstellung monoklonaler Antikörper zurückgreifen.

Erfindungsgemäße Inhibitoren sind solche niedermolekularen oder aber auch proteinartige Substanzen, die die Protein-Kinase-Aktivität des erfindungsgemäßen Proteins selektiv hemmen bzw. vollkommen ausschalten können. Das Identifizieren und Herstellen geeigneter Inhibitoren ist dem Fachmann unter Verwendung herkömmlicher Protein-Kinase-Assays zum Screenen von Substanzen ohne Weiteres möglich. Geeignete Screeningverfahren und Protein-Kinase-Assays werden unten beschrieben. Auch lassen sich geeignete Substanzen mit gewünschter Bindungsaffinität unter Einsatz der Computer-gestützten Wirkstoffentwicklung (CAD) identifizieren (Vergleich z.B. Böhm, Klebe, Kubinyi, 1996, Wirkstoffdesign, Spektrum-Verlag, Heidelberg). Die so identifizierten Inhibitoren der erfindungsgemäßen Protein-Kinase sind beispielsweise geeignet zur Prophylaxe und/oder Therapie des Schlaganfalls und anderer neurologischer (insbesondere neurodegenerativer) oder neoplastischer Erkrankungen. Für die genannten niedermolekularen Substanzen kommen als Inhibitoren auch Peptide und Proteine in Betracht, die eine spezifische Wechselwirkung mit dem erfindungsgemäßen Protein-Kinase-Inhibitor eingehen können. Solche Peptid- bzw. Proteininhibitoren lassen sich beispielsweise mit Hilfe des Two-Hybrid-Systems oder aber auch anderen Assays identifizieren. Diese Assays erlauben die Eingrenzung der Aminosäuren, die für eine spezifische Wechselwirkung mit weiteren Interaktionspartnern verantwortlich sind. Ferner lässt sich das erfindungsgemäße Protein und dessen Protein-Kinase-Aktivität einfach in einem Testsystem austesten, wobei die Aktivität des Proteins in Anwesenheit der zu testenden Substanz gemessen wird. Hierbei handelt es sich vorzugsweise um einfache Meßmethoden (kolorimetrische, luminometrische, auf Fluoreszenz beruhende oder radioaktive Techniken), die die schnelle Messung einer Vielzahl von Testsubstanzen erlauben (vergleiche Böhm, Klebe, Kubinyi, 1996, Wirkstoffdesign, Spektrum-Verlag, Heidelberg). Die beschriebenen Testsysteme erlauben das Durchsuchen von chemischen Bibliotheken nach Substanzen, die hemmende oder aber auch aktivierende Wirkungen auf erfindungsgemäße Proteine haben. Mit diesen Inhibitoren lässt sich die Signaltransduktionskette, die bei Ischämie induziert wird und über das erfindungsgemäße Protein verläuft, hemmen. Dies ermöglicht das Hemmen bzw. Verhindern der ischämischen Folgeerscheinungen.

Mittels des oben erwähnten Two-Hybrid-Selektions-Systems lassen sich auch die Intrazellulären physiologischen Interaktionspartner der erfindungsgemäßen Protein-Kinase, wie das Phosphorylierungssubstrat, sowie die Kinase-Aktivität regulierende intrazelluläre Interaktionspartner identifizieren.

Mit Hilfe von Antikörpern kann die Proteinmenge und auch die Aktivität (z. B. spezifische Phosphorylierungen) der Proteine mit den Sequenzen der SEQ ID Nr. 5-8 bestimmt werden. Daher ist ein weiterer Gegenstand der Erfindung ein Verfahren zur Quantifizierung der Proteinaktivität eines Proteins mit einer der Sequenzen SEQ ID Nr. 5-8. Ausgehend von den Aminosäuresequenzen nach SEQ ID Nr. 5-8 können synthetische Peptide generiert werden, die als Antigene für die Produktion von Antikörpern eingesetzt werden. Es ist auch möglich, das Polypeptid oder Bruchstücke davon zur Generierung von Antikörpern einzusetzen. Mit "Antikörpern" sind sowohl polyklonale, monoklonale, humane oder humanisierte oder rekombinante Antikörper oder Fragmente davon, single chain Antikörper oder auch synthetische Antikörper gemeint. Unter erfindungsgemäßen Antikörpern oder deren Fragmenten sind prinzipiell alle Immunoglobulinklassen wie IgM, IgG, IgD, IgE, IgA oder ihre Subklassen wie die Subklassen des IgG oder deren Mischungen zu verstehen. Bevorzugt sind IgG und seine Subklassen wie beispielsweise IgG1, IgG2, IgG2a, IgG2b, IgG3 oder IgGM. Besonders bevorzugt sind die IgG Subtypen IgG1/k oder IgG2b/k. Als Fragmente seien alle verkürzten oder veränderten Antikörperfragmente mit einer oder zwei dem Antigen-komplementären Bindungsstellen, wie Antikörperteile mit einer den Antikörper entsprechenden von leichter und schwerer Kette gebildeten Bindungsstelle wie Fv-, Fab- oder F(ab')2-Fragmente oder Einzelstrangfragmente, genannt. Bevorzugt sind verkürzte Doppelstrangfragmente wie Fv-, Fab- oder F(ab')2. Diese Fragmente können beispielsweise auf enzymatischem Wege durch Abspaltung des Fc-Teils der Antikörper mit Enzymen wie Papain oder Pepsin, durch chemische Oxidation oder durch gentechnische Manipulation der Antikörpergene erhalten werden. Auch genmanipulierte nichtverkürzte Fragmente können vorteilhaft verwendet werden. Die Antikörper oder Fragmente können allein oder in Mischungen verwendet werden. Die Antikörpergene für die gentechnischen Manipulationen lassen sich in dem Fachmann bekannter Weise, beispielsweise aus den Hybridomzellen, isolieren (Harlow, E. and Lane, D. 1988, Antibodies: A Laboratory Manual, Cold Spring Harbor Press, N.Y.; Ausubel et al., (eds), 1998, Current Protocols in Molecular Biology, John Wiley & Sons, New York). Dazu werden Antikörper-produzierende Zellen angezogen und die mRNA bei ausreichender optischer Dichte der Zellen über Zellyse mit Guanidiniumthiocyanat, Ansäuern mit Natriumacetat, Extraktion mit Phenol, Chloroform/Isoamylalkohol, Fällungen mit Isopropanol und Waschen mit Ethanol aus den Zellen in bekannter Weise isoliert. Anschließend wird mit Hilfe der Reversen Transcriptase cDNA aus der mRNA synthetisiert. Die synthetisierte cDNA kann direkt oder nach genetischer Manipulation beispielsweise durch "site directed mutagenesis", Einführung von Insertionen, Inversionen, Deletionen oder Basenaustausche in geeignete tierische, pilzliche, bakterielle oder virale Vektoren inseriert und in den entsprechenden Wirtsorganismen exprimiert werden. Bevorzugt werden bakterielle oder Hefe-Vektoren wie pBR322, pUC18/19, pA-CYC184, Lambda oder Hefe-mu-Vektoren zur Klonierung der Gene und die Expression in Bakterien wie E. coli bzw. in der Hefe wie Saccharomyces cerevisiae. Spezifische Antikörper gegen die erfindungsgemäßen Proteine können sich sowohl als diagnostische Reagenzien als auch als Therapeutika bei Erkrankungen eignen, bei denen das erfindungsgemäße Protein von pathophysiologischer Bedeutung ist.

Der erfindungsgemäße Diagnosekit enthält eine der erfindungsgemäßen Nukleinsäuren, oder ein Konstrukt enthaltend diese, ein erfindungsgemäßes Protein und/oder einen Antikörper, der für das erfindungsgemäße Protein spezifisch ist, sowie die weiteren Reagenzien, die üblicherweise Bestandteil diagnostischer Kits sind. Hierzu zählen geeignete Pufferlösungen und weitere Nachweisreagenzien.

Bei dem erfindungsgemäßen Verfahren zur Diagnose eines Apoplexierisikos oder der Verlaufsbeurteilung eines Himinfarktes wird die Patientenprobe mit einer erfindungsgemäßen Nukleinsäure in Kontakt gebracht und die damit hybridisierende Nukleinsäure in der Patientenprobe bestimmt. Ein erhöhter Spiegel an Nukleinsäure, die mit der erfindungsgemäßen Nukleinsäure hybridisiert ist ein Indikator für ein erhöhtes Risiko des Auftretens einer Apoplexie. Alternativ zu dem Nachweis von Nukleinsäure in der Patientenprobe kann auch die Menge an erfindungsgemäßem Protein als Indikator eines Apoplexierisikos bestimmt werden. Für den Proteinnachweis können beispielsweise die erfindungsgemäßen Antikörper herangezogen werden. Ein erhöhter Proteinspiegel in der untersuchten Patientenprobe ist ebenfalls ein Indikator für ein erhöhtes Apoplexierisiko. Diese Bestimmungen sind auch ohne weiteres quantitativ durchführbar, wobei die Negativkontrolle Material eines gesunden Patienten darstellt.

Ferner können die erfindungsgemäße Nukleinsäure sowie das davon kodierte Protein bzw. Oligonukleotide und Peptide hiervon sowie dagegen gerichtete Antikörper zur Diagnose weiterer Erkrankungen, insbesondere neurologischer oder kardiovaskulärer oder immunologischer oder Tumorerkrankungen, verwendet werden. Ferner können diese Materialien für die Diagnose von genetischen Prädispositionen für bestimmte neurologische, neoplastische, kardiovaskuläre und immunologische Erkrankungen herangezogen werden. Weiterhin lässt sich mit diesen Materialien ein Monitoring der Behandlung der genannten Erkrankungen durchführen.

Ein Verfahren zum qualitativen und quantitativen Nachweis einer erfindungsgemäßen Nukleinsäure in einer biologischen Probe, umfasst folgende Schritte: a) Inkubation einer biologischen Probe mit einer bekannten Menge an erfindungsgemäßer Nukleinsäure oder einer bekannten Menge an Oligonukleotiden, die als Primer für eine Amplifikation der erfindungsgemäßen Nukleinsäure geeignet sind, b) Nachweis der erfindungsgemäßen Nukleinsäure durch spezifische Hybridisierung oder PCR-Amplifikation, c) Vergleich der Menge an hybridisierender Nukleinsäure oder an durch PCR Amplifikation gewonnener Nukleinsäure mit einem Mengenstandard. Ein Verfahren zum qualitativen und quantitativen Nachweis eines erfindungsgemäßen Proteinheteromers oder eines erfindungsgemäßen Proteins in einer biologischen Probe umfasst folgende Schritte: a) Inkubation einer biologischen Probe mit einem Antikörper, der spezifisch gegen das Proteinheteromer oder gegen das erfindungsgemäße Protein gerichtet ist, b) Nachweis des Antikörper / Antigenkomplexes, c) Vergleich der Mengen des Antikörper/Antigenkomplexes mit einem Mengenstandard. Als Standard wird üblicherweise eine biologische Probe aus einem gesunden Organismus entnommen. Insbesondere kann hier die unten ausgeführte Eigenschaft der Nukleinsäure gemäß SEQ ID Nr. 1 - 4 benutzt werden, nach bestimmten pathophysiologischen Stimuli, wie z.B. cerebralen I-schämien, hochreguliert zu werden. Dies betrifft z.B. die Verlaufsbeurteilung von Krankheiten (wie z.B. des Schlaganfalls), die Beurteilung von Therapieerfolgen, die Graduierung einer Krankheit.

Die erfindungsgemäße pharmazeutische Zusammensetzung enthält eine erfindungsgemäße Nukleinsäure, ein Konstrukt enthaltend diese, eine Wirtszelle enthaltend die genannten Gegenstände, ein erfindungsgemäßes Protein, einen dagegen gerichteten Antikörper und/oder einen erfindungsgemäßen Inhibitor gegebenenfalls zusammen mit den üblichen Hilfs- und Trägerstoffen.

Therapeutische Anwendungen der erfindungsgemäßen Gegenstände betreffen die Modulation von Prozessen, die mit der Phosphorylierung von körpereigenen Proteinen zusammenhängen. Hierzu zählen auch die folgenden physiologischen bzw. pathophysiologischen Vorgänge: Beeinflussung immunologischer Aktivierungsprozesse (z.B. Aktivierung von Monozyten, T-Zellen); die Beeinflussung von Zelltodprozessen, z.B. Kaskaden, die zu Zelltod führen, oder von Prozessen, die zu ungehemmtem Wachstum führen. Zu den behandelbaren Zelltypen zählen insbesondere neurale Zellen, Tumorzellen und Zellen des Immunsystems. Schließlich lässt sich mit den erfindungsgemäßen Materialien die Zellinteraktionen, an denen Protein-Kinasen beteiligt sind, z.B. die Zellteilung, Zelldifferenzierung, Plastizität und Regeneration beeinflussen.

Die erfindungsgemäße Nukleinsäure sowie das Konstrukt enthaltend diese, die entsprechenden wirtszellen sowie das erfindungsgemäße Protein, der erfindungsgemäße Antikörper und/oder der erfindungsgemäße Inhibitor können insbesondere zur Prophylaxe und/oder Therapie neurologischer, insbesondere neurodegenerativer Erkrankungen eingesetzt werden. Hierzu zählen insbesondere Schlaganfall, Multiple Sklerose, Morbus Parkinson, Amyotrophe Lateralsklerose, Heterodegenerative Ataxien, Morbus Huntington, Neuropathien und Epilepsien.

Ebenso können bevorzugt damit Tumorerkrankungen diagnostiziert/therapiert werden. Beispiele für solche Tumorerkrankungen sind z.B. Kolonkarzinome, Rhabdomyosarkome, Bronchialkarzinome.

Die erfindungsgemäßen Nukleinsäuren lassen sich auch im Rahmen der Gentherapie bei Säugern und insbesondere den Menschen einsetzen. Bei der Gentherapie können die erfindungsgemäßen Sequenzen entweder in den Körper oder Teile davon eingebracht werden oder die Expression der körpereigenen Sequenzen kann reguliert werden, wie beispielsweise mittels der Antisensetechnologie. Zu diesem Zweck können Oligonukleotide, z.B. mit Antisinnorientierung oder Hybrid-RNA-DNA-Oligonukleotide, die erfindungsgemäße Sequenzen enthalten, benutzt werden. Ferner können virale Konstrukte, enthaltend eine erfindungsgemäße Sequenz, eingesetzt werden. Schließlich kann nackte DNA, enthaltend eine erfindungsgemäße Nukleinsäure, eingesetzt werden.

Die in den erfindungsgemäßen Nukleinsäuren identifizierten SNPs sind ebenfalls in der Diagnose unter Erforschung humaner Erbkrankheiten nützlich. Die erfindungsgemäßen Nukleinsäuren können dazu verwendet werden, weitere Gene für homologe mRNAs im murinen und im menschlichen Genom mit gängigen Methoden durch Homologiescreening zu isolieren und zu charakterisieren und mit bereits bekannten Markem für humane Erbkrankheiten zu korrelieren. Dies ermöglicht die Identifizierung weiterer Gene als Ursache für bestimmte Erbkrankheiten.

### Figurenbeschreibung

- Fig. 1: zeigt das Prinzip des Restriction-mediated Differential Display (RMDD)-Verfahrens.
- Fig. 2: Gewebeverteilung des erfindungsgemäßen Proteins in der Ratte:
Die obere Abbildung zeigt einen Northern-Blot der mit einer Maussonde aus dem 3'-Bereich der SEQ ID Nr. 1 erhalten wurde. Die untere Abbildung zeigt die Ethidiumbromidfärbung des im oberen Teil gezeigten RNA-Gels.
Es wird eine einzige Bande von der Probe detektiert. Die weitaus stärkste Expression des erfindungsgemäßen Proteins zeigt sich im Gehirn; in Testis und Lunge finden sich ebenfalls noch deutliche Transkripte, während die anderen Gewebe nur sehr schwache Banden aufweisen.

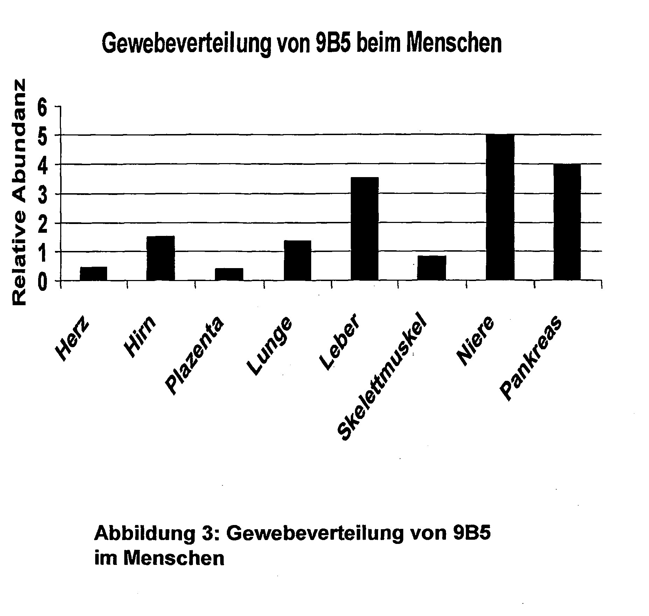

- Fig. 3: zeigt die Gewebeverteilung des erfindungsgemäßen Proteins beim Menschen.
Gezeigt ist eine quantitative PCR mit Hilfe des Light-Cycler-Systems und der Benutzung des MTC (multiple tissue cDNA) Kits der Firma Clontech. Die stärkste Expression befindet sich im Gehirn und der Lunge und den Intestinalorganen.

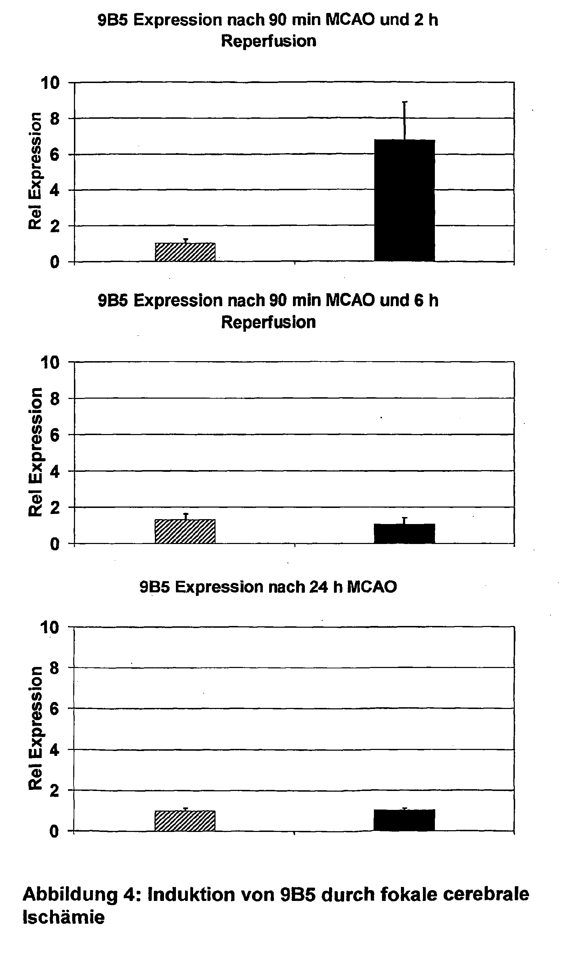

- Fig. 4: zeigt die Induktion des erfindungsgemäßen Proteins durch fokale cerebrale Ischämie.
Gezeigt ist die Quantifizierung von 9B5 mRNA mit Hilfe des Light-Cycler-Systems nach Induktion einer fokalen cerebralen Ischämie an Mäusen. Die erfindungsgemäße Nukleinsäure wird zwei Stunden nach einer ischämischen Episode hochreguliert. Sechs Stunden nach Ischämie ist diese Induktion nicht mehr nachweisbar.

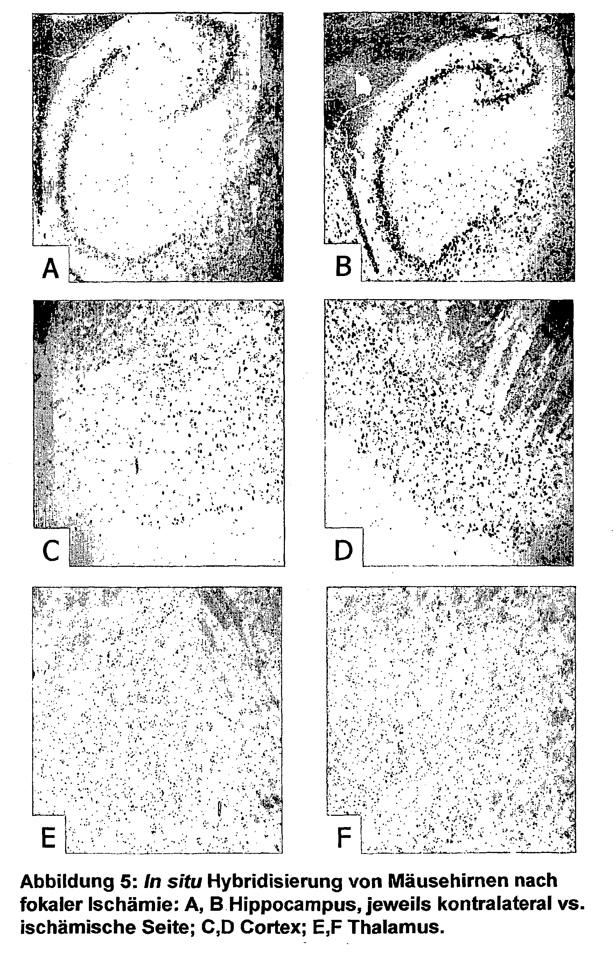

- Fig. 5: zeigt die Induktion des erfindungsgemäßen Proteins durch fokale cerebrale Ischämie (in situ Hybridisierung von Mausgehim)
Gezeigt ist eine in situ-Hybridisierung von 9B5 nach Induktion einer fokalen cerebralen Ischämie an Mäusen. Die erfindungsgemäße Nukleinsäure wird zwei Stunden nach einer ischämischen Episode hochreguliert (linke = ischämische = induzierte Seite). Man sieht ausserdem, dass 9B5 in Neuronen exprimiert wird. Besondere Induktion findet man im Kortex und im hippocampalen Gebiet.

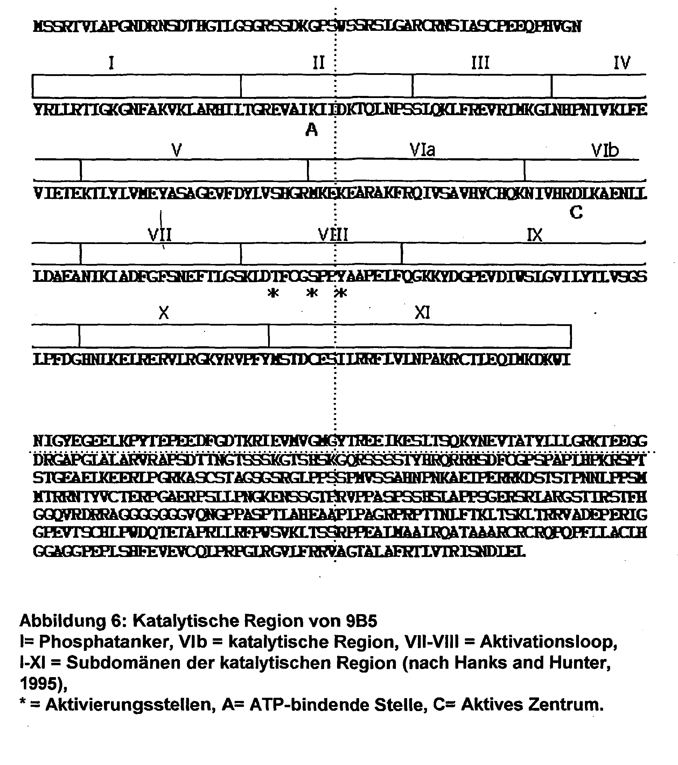

- Fig. 6: zeigt die katalytische Region der SEQ ID Nr. 7. Dabei besitzen die Symbole folgende Bedeutung: I = Phosphatanker, VIb = katalytische Region, VII - VIII = Aktivierungsloop, V-XI = katalytische Domäne mit Subdomänen (nach Hanks und Hunter, 1995, FASEB J., 9, 576-596); * = Aktivierungsstelle, A = ATP-Bindungsstelle; C = aktives Zentrum.

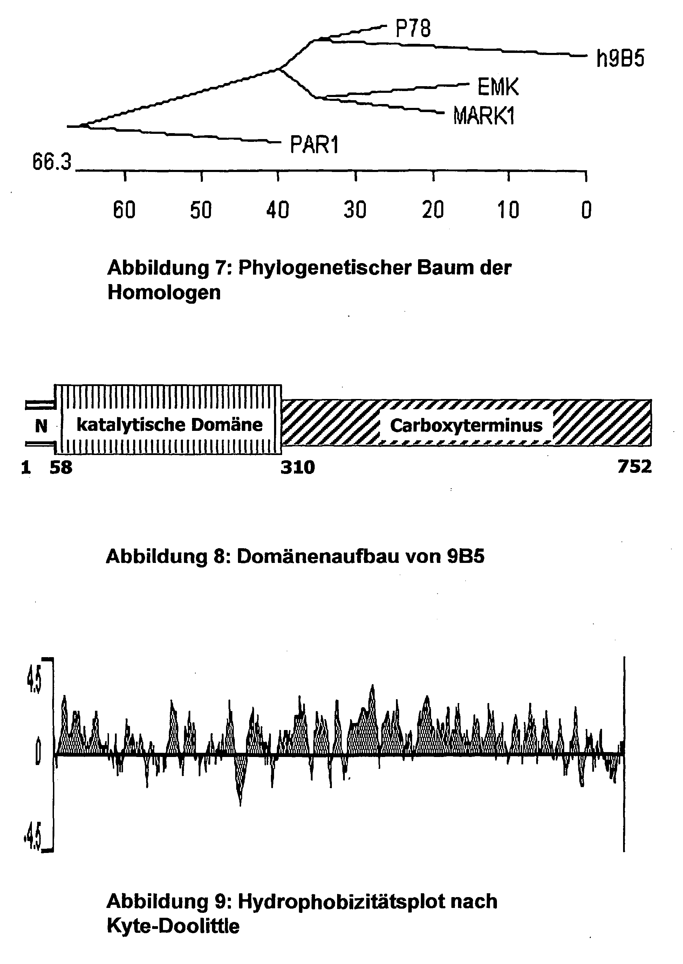

- Fig. 7: zeigt den physiologischen Baum zu homologen Protein-Kinasen. H9B5 ist das phylogenetisch jüngste Protein und am engsten verwandt zu P78/c-TAK 1. Klar gezeigt ist auch die frühe Abspaltung des C.elegans-Proteins Par-1.
- Fig. 8: zeigt den Domänenaufbau von 9B5.
- Fig. 9: ist ein Hydrophobizitätsblot nach Kyte-Doolittle.

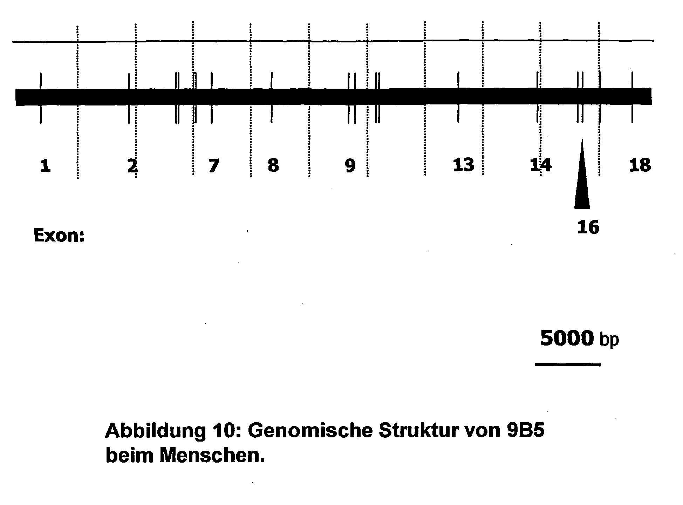

- Fig. 10: zeigt die genomische Struktur von 9B5 beim Menschen.
Gezeigt sind die 18 Exone des 9B5-Gens. Exon 16 unterliegt differenziellem Spleißen. Die Variante 9B5 entsteht ohne Exon 16; die Variante 9B5A enthält Exon 16.

### Proteinsequenz von 9B5

Die murinen Proteinsequenzen (SEQ ID Nr. 5 und 6) sind am 5' Ende unvollständig. Bei den humanen Sequenzen gemäß SEQ ID Nr. 7 und 8 handelt es sich um Voll-längen-Klone. Dies ergibt sich auch aus Alignments mit homologen Protein-Kinasen.

Für h9B5 (SEQ ID Nr. 7) ergibt sich ein offenes Leseraster, das für ein Protein mit 752 Aminosäuren kodiert (82.5 kD Molekulargewicht; isoelektr. Punkt bei pH 9,7). Das Programm PSORT II ergibt keine signifikanten Signalsequenzen, und sagt keine Transmembranbereiche vorher. Ein Kyte-Doolittle Hydrophobizitätsplot (Fig. 9) zeigt ebenfalls einen hohen Anteil an hydrophilen Bereichen und nur einen grösseren hydrophoben Bereich (ca. AS 240-255), der jedoch nicht ausreichend hydrophob für eine Transmembranregion scheint (Index > -3). Interessant ist jedoch in diesem Zusammenhang die berichtete membranassoziierte Lokalisation von einigen verwandten Proteinen (p78/c-TAK1, par1).

Für die Sequenz h9B5_b (SEQ ID Nr. 8) ergibt sich ein offenes Leseraster von 688 A-mionsäuren mit einem Molekulargewicht von 75,3 kD und einem isoelektrischen Punkt bei pH 9,8. Durch die Insertion von Exon 16 findet eine Rasterverschiebung statt, die zu einem früheren Translationsstopp führt.

### Klassifizierung von 9B5

Ein Vergleich mit bekannten Protein-Kinasen erlaubt die Zuordnung des erfindungsgemäßen Proteins zu der Subgruppe "CaMK Gruppe II" nach Hanks mttp://www.sdsc.edu/Kinases/pkr/pk catalytic/pk hanks seq align long.html), (Vergleich Hanks and Lindberg, Methods Enzymol, 200, 525-32, (1991); Hanks and Hunter, Faseb J, 9, 576-96, (1995)) ). Drewes et al. *(*Drewes, et al., Cell, 89, 297-308, (1997)) postulierten bereits für MARK1/MARK2 und p78 eine neue Subgruppe in der bereits definierten SNF1/AMPK Subgruppe der CaMKII Gruppe. Nach dem Alignment der am stärksten homologen Proteine (9B5, Par1, cTAK1/p78, MARK1, EMK) und dem phylogenetischen Baum (Fig. 7) besteht die Möglichkeit, dass 9B5 eine neue Subgruppe definiert. Am Carboxyterminus weicht 9B5 stark von den anderen Protein-Kinasen ab. Die in dieser Gruppe befindlichen Kinasen zeichnen sich durch eine stark konservierte carboxyterminale Domäne aus, die mit der Sequenz "ELKL" endet. Deshalb wurde EMK auch als "ELKL-motif kinase" bezeichnet. Es ist davon auszugehen, dass noch Protein-Kinasen existieren, die 9B5 im Carboxyterminus ähnlich sind, und die sich über Homologiescreens identifizieren lassen. Eine Suche mit dem Programm "tblastn" gegen die EMBL-Nucleotiddatenbank ergab bislang keine Hinweise auf bereits bekannte Homologe im Carboxyterminusbereich.

Weiter ist aus der Veröffentlichung WO9801756 die Protein Kinase TcAK1 mit etwa 67 % Identität in der codierenden Region mit 9b5 (SEQ ID NO:1) bekannt.

Das physiologische Substrat des erfindungsgemäßen Proteins können Proteine mit der Zielsequenz KXGS und/oder KVGS sein. Demzufolge kann das Tauprotein oder die Proteine MAP1 und 2 ein physiologisches Substrat der erfindungsgemäßen Protein-Kinase darstellen.

### Subdomänen der katalytischen Domäne: 9B5 ist eine Serin-Threonin-Protein-Kinase

Eindeutig lassen sich über Vergleiche mit anderen Protein-Kinasen die wichtigen katalytischen Subdomänen definieren (siehe Fig. 6). Ebenso lassen sich die Aktivierungsstellen aufzeigen (ein Threonin und ein Serin die phosphoryliert werden können; pos. 211 und 215 in h9B5, Fig. 6). Eine Mutation dieser Aminosäuren z.B. zu Alanin führt zu einer konstitutiven Inaktivierung. Ebenso führt die Mutation des Lysinrestes (ATP-Bindung) in Subdomäne II (siehe Fig. 6) zu einer Inaktivierung. Diese Kenntnis kann für weitere Experimente benutzt werde, z.B. unter Benutzung dominant negativer Effekte. Eine Mutation von Pos. 211 (Ser) und 215 (Thr) zu Glutamat oder Aspartat sollte zu konstitutiver Aktivierung führen, da die negativ geladenen Gruppen eine Phosphorylierung imitieren können *(*Huang and Erikson, Proc Natl Acad Sci U S A, 91, 8960-3, (1994)*)*. Diese Mutanten können für Überexpression der spezifischen Kinase-Aktivität in Zellen oder transgenen Tieren ausgenutzt werden.

Von Subdomäne I ist bekannt, dass sie als eine Art Klammer wirkt, die die nichttransferierbaren Phosphatgruppen von ATP verankert. Dabei passt die Peptidsequenz GKGNFAKV in 9B5 gut zum Konsensusmotiv GXGXXGXV *(*Hanks and Hunter, Faseb J, 9, 576-96, (1995)*).* In der Subdomäne II ist v.a. das Lysin (markiert mit "A" in Fig. 6) wichtig; dieses verankert und orientiert die alpha- und beta-Phosphate von ATP und ist essentiell für die Enzymfunktion. Subdomäne Vlb ist durch die Konsensussequenz HRDLKXXN charakterisiert. Diese ist auch in 9B5 sehr gut konserviert: HRDLKAEN.
Dabei ist das Aspartat (D) wahrscheinlich der Protonenakzeptor für die angreifende Hydroxylgruppe während der Phosphotransferreaktion *(*Hanks and Hunter, Faseb J, 9, 576-96, (1995)). Subdomäne VII bildet mit Mg2+-lonen einen Chelatkomplex, der das gamma-Phosphat einschliesst und somit diese Gruppe für den Transfer orientiert. Praktisch invariabel ist die Sequenz DFG. Ebenso erhalten ist die konservierte Sequenz APE in Subdomäne VIII. Diese Domäne ist für die Peptiderkennung zuständig. Hier können auch Inhibitorpeptide binden.Viele Protein-Kinasen werden durch Phosphorylierung innerhalb dieser Subdömäne aktiviert. Diese Domäne ist vollständig konserviert in EMK, p78 und MARK1. Bei MARK1 wurde durch direkte Sequenzierung bereits gezeigt, dass Threonin (T) und Serin (S) phosphoryliert werden können, und MARK1 dadurch aktiviert wird. Es kann wegen dieser Konservierung mit höchster Wahrscheinlichkeit davon ausgegangen werden, dass 9B5 ebenfalls an diesen Stellen phosphoryliert und damit reguliert werden kann. Möglicherweise findet Autophosphorylierung statt, eine Aktivierung durch eine andere Kinase ist auch denkbar. Möglicherweise ist auch das Tyrosin (Y) in dieser Domäne eine Phosphorylierungsstelle (wie z.B. bei Erk1/2). Subdomäne IX ist ebenfalls an der Peptidbindung beteiligt (hydrophobe Wechselwirkung).
Aufgrund dieser Strukturentwicklung kann mit Sicherheit davon ausgegangen werden, dass 9B5 eine aktive Protein-Kinase (Serin-Threonin-Kinase) ist.

### Gewebeexpression von 9B5

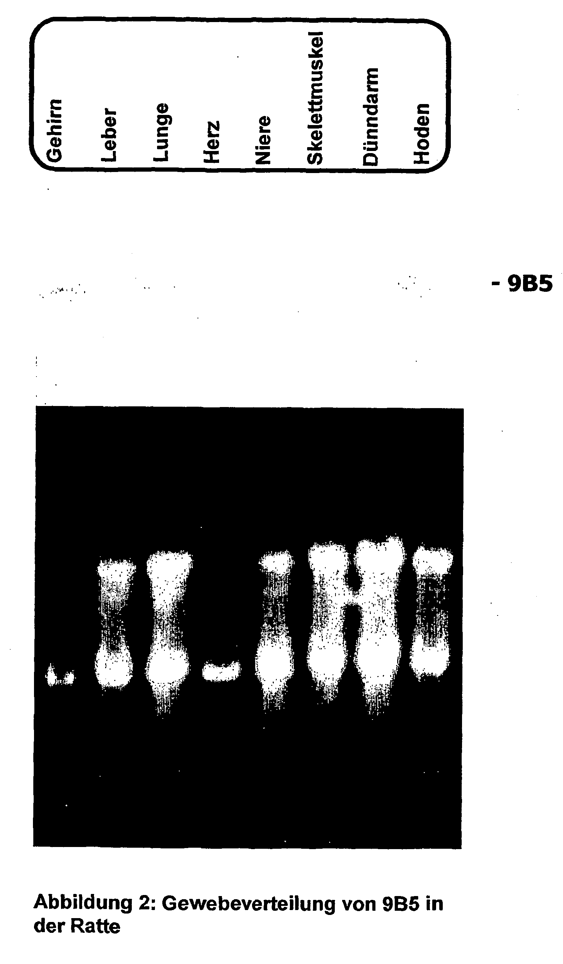

Die Gewebeexpression von 9B5 wurde bei der Maus und beim Menschen untersucht. Bei der Maus wurde ein "multiple tissue northern" mit verschiedenen Geweben durchgeführt (Fig. 2). Als Probe wurde ein Fragment aus dem 3' Bereich der Maus cDNA verwendet. Hier findet sich Expression in Gehirn, Hoden und Lunge.

Beim Menschen wurde initial ein "multiple tissue northern" (Clontech) mit 2 verschiedenen Sonden (aus dem 3' und aus dem 5' Bereich) durchgeführt. Es war jedoch kein deutliches Signal zu erhalten, was auf das geringe Vorkommen von 9B5 beim Menschen zurückgeführt wurde. Deshalb wurde eine quantitative PCR mit Hilfe des Light-Cyclers (Roche Diagnostics, Mannheim) durchgeführt. Es wurden cDNA-Proben von 8 humanen Geweben verwendet, die bereits mengenstandardisiert auf 4 verschiedene Housekeeping Gene sind (Fa. Clontech). Als Kontrolle verwendet wurde Plasmid h9B5-663, das ein amplifiziertes Fragment humaner 9B5-cDNA enthielt. Als PCR-Programm wurde ein touchdown-Protokoll gewählt. Es fand in allen Gewebeproben Amplifikation eines Produktes mit dem Schmelzpunkt 90°C statt, dieser deckte sich mit der Kontroll-PCR. Eine darauffolgende Gelanalyse der Fragmente erbrachte ein Produkt von ca. 660 bp, also die erwartete Größe. 9B5 beim Menschen ist tatsächlich nicht sehr stark exprimiert, die Amplifikation wird im LightCycler erst um Zyklus 32 sichtbar. Folgende Primer wurden verwendet:
seq_h9b5_s1 GTTGCCATCAAGATTATC (in Exon 3)
seq_h9b5_a4 CATGATTTGCTCGAGAGTAC (in Exon 9)
Die quantitative Analyse (Fig. 3) zeigt eine relativ ubiquitäre Gewebeverteilung mit Schwerpunkten in Gehirn und Lunge, Leber, Niere und Pankreas.

### Regulation durch fokale cerebrale Ischämie, einem Schlaganfallmodell

Die erfindungsgemäße Serin-Threonin-Kinase 9B5 wurde durch ein Verfahren zur Klonierung differentiell regulierter Gene (RMDD) in der ischämischen Hemisphäre von Mäusen nach fokaler cerebraler Ischämie identifiziert. Das Tiermodell der fokalen cerebralen Ischämie stellt ein valides Modell für den humanen ischämischen Schlaganfall dar. Zur Herbeiführung der fokalen cerebralen Ischmämie wurde das sog. Fadenmodell benutzt, bei dem ein beschichteter Nylonfaden durch die A. carotis interna an den Abgang der A. cerebri media vorgeschoben wird und einen ischämischen Schlaganfall induziert *(*Clark, et al., Neurol. Res., 19, 641-648, (1997)*).* Die Regulation der Genexpression spielt bei der cerebralen Ischämie eine entscheidende Rolle für den Ablauf und das Ausmass des Neuronenschadens *(Koistinaho and Hokfelt, Neuroreport, 8, i-viii, (1997,* Schneider, et al., Nat Med, 5, 554-9, (1999)*)*. Insbesondere "immediate early" Gene spielen hier eine Rolle *(*Atkins, et al., Stroke, 27, 1682-1687, (1996)*),* wie z.B. cox-2, *(*Nogawa, et al., J. Neurosci., 17, 2746-2755, (1997)*).*

Die 9B5 Expression wurde nach einer fokalen cerebralen Ischämie in drei Zeitverläufen untersucht: Zum einen in einer transienten Ischämie nach zwei Reperfusionszeiten (90 min Ischämie, 2 h und 6 h Reperfusion), und zum anderen in einer permanenten Ischämie von 24 h (Fig. 4). RNA wurde aus den beiden Hemisphärenhälften von 3-4 Gehirnen ohne Hirnstamm und Kleinhim gewonnen (Fasttrack kit, Invitrogen). Mit Hilfe des LightCycler^{™} Systems (Roche Diagnostics, Mannheim) wurde eine quantitative PCR durchgeführt. Der cDNA Gehalt der Proben wurde auf die Expression von Cyclophilin und S20 normiert *(*Schneider, et al., Proc Natl Acad Sci U S A, 92, 4447-51, (1995)*)*. Benutzte Primer zur Amplfikation von Cyclophilin waren:
cyc5 ACCCCACCGTGTTCTTCGAC
acyc300 CATTTGCCATGGACAAGATG
und zur Amplifikation von Maus 9B5:
9_B5_(1)_1s TATGATCGAACCTCCTTCATGCC
9_B5_(1)_1a ATGTCCAGAACTGGGCCTAGCG (Diese Primer amplifizieren ein Amplimer von 556 bp, das am 3' Ende der Maus-cDNA liegt).

Tatsächlich zeigt sich eine deutliche Hochregulation um den Faktor 7-8 von 9B5-RNA auf der ischämischen (linken) Hirnhälfte 2h nach dem ischämischen Ereignis (middle cerebral artery occlusion von 90 min und 2 h Reperfusion; (Fig. 4); die Fehlerbalken zeigen Standardabweichungen, diese ergeben sich aus Messungen mit 3-fach seriell verdünnten cDNA-Proben und spiegelt damit die Zuverlässigkeit der Messergebnisse dar). Nach 24 h (in einem permanenten Modell) hingegen konnte kein Unterschied mehr festgestellt werden.

Ebenfalls durchgeführt wurde eine *in-situ*-Hybridisierung mit einer 1,6 kb langen Sonde aus dem 3' Bereich der Maus-9B5-cDNA (jeweils sense und antisense). Es wurde dabei im Wesentlichen nach dem Protokoll der Firma Roche Diagnostics (Digoxigeninsystem) nach Modifikationen von Rossner et al. (Mol Cell Neurosci, 9, 460-475, (1997)) verfahren. Es zeigt sich eine deutliche Induktion auf der ischämische Seite (links) besonders in Neuronen der hippocampalen Region und des Cortex, geringer im Thalamus (Fig. 5).

Ebenso wird deutlich, dass 9B5 überwiegend in Neuronen exprimiert wird, und dort einer Regulation unterliegt.

Dies zeigt, dass 9B5 eine wichtige Rolle in der Pathogenese des Schlaganfalls spielt. Die Hochregulation von 9B5 hierbei eine ähnliche Rolle wie z.B. bei bekannten Serin-Threonin-Kinasen (z.B. JNK, p38).

Eine Vielzahl an Serin-Threonin-Kinasen sind an Zelltodprozessen beteiligt (z.B. ASK1 *(*Tobiume, et al., Biochem Biophys Res Commun, 239, 905-10*, (*1997, Berestetskaya, et al., J Biol Chem, 273, 27816-23*, (1998,* Chen, et al., Oncogene, 18, 173-80, (1999)*),* DAP *(*Inbal, et al., Nature, 390, 180-4*, (1997,* Levy-Strumpf and Kimchi, Oncogene, 17, 3331-40, (1998)*)*, DRAKs *(*Sanjo, et al., J Biol Chem, 273, 29066-71, (1998*))* , ZIP *(*Kawai, et al., Mol Cell Biol, 18, 1642-51, (1998)*)* ), DRP-1 *(*Inbal, et al., Mol Cell Biol, 20, 1044-54, (2000)*).*

Diese Hochregulation ist ein Beweis für einen neuen transkriptionellen Regulationsmechanismus bei Protein-Kinasen, und bislang das einzige bekannte Beispiel dafür im Säugetiersystem. Interessanterweise fand sich erst jüngst die Hochregulation mehrerer MAP-Kinasen in einer systematischen Untersuchung des Hefetranskriptoms *(*Roberts, et al., Science, 287, 873-80, (2000)*)*. Dies könnte einen neuen generellen Mechanismus zur Regulation von Protein-Kinasen darstellen.

### Pharmakologische Bedeutung von 9B5 in neurodegenerativen, neoplastischen und anderen Erkrankungen

In jüngerer Zeit gewinnen Ansätze zur Inhibierung / Beeinflussung von Signaltransduktionswegen "downstream" eines membranständigen Rezeptors in der pharmakologischen Forschung zunehmend Bedeutung. Diese Ansätze werden wahrscheinlich in Zukunft einen wichtigen Anteil in der Therapie humaner Erkrankungen haben, insbesondere bei bis jetzt schlecht oder nicht therapierbaren Erkrankungen *(*Kletsas and Papavassiliou, Exp. Opin. Invest. Drugs, 8, 737-746, (1999)*).* Vorteile dieser Ansätze sind, dass sie erstens Ereignisse beeinflussen können, die von mehreren Stimuli hervorgerufen werden können, und eine gemeinsame Endstrecke haben; zweitens, dass zelluläre Ereignisse zeitlich nach dem auslösenden Stimulus liegen, und deshalb länger einer Intervention zugänglich sind.

Beispiele für erfolgreiche Eingriffe in solche Signalkaskaden sind beispielsweise Inhibitoren für Caspasen, die Apoptoseprozesse noch längere Zeit nach einem auslösenden Stimulus blockieren können. Besondere Aufmerksamkeit hat auch der Transkriptionsfaktor NF-kappaB und diesen aktivierende Prozesse gefunden. Beispielsweise wurde die Klonierung der 1-kappaB-Kinasen mit dem Ziel verfolgt, spezifische Inhibitoren für NF-kappaB vermittelte Gentranskription zu finden. Differentielle Transkriptionsprofilierungen gewinnen in der Pharmaforschung zur Identifizierung möglicher neuer Ansatzpunkte für Pharmaka in letzter Zeit grosse Bedeutung. Transkriptionelle Regulation, d.h. Regulation der mRNA-Menge eines Gens in der Zelle, ist ein wesentlicher Schritt für die Reaktion der Zelle auf Stimuli, neben Proteinphosphorylierungen, Proteindegradation etc. 9B5 unterliegt offensichtlich einer sehr raschen Regulation durch transkriptionelle Aktivierung, wie oben gezeigt wurde. Oft sind solche schnell regulierten Gene an kritischen Schlüsselstellungen für zelluläre Vorgänge beteiligt.

Eine pharmakologische Beeinflussung von 9B5 ist wie folgt möglich: 1. eine Beeinflussung der Transkriptmenge in der Zelle, beispielsweise die Suppression der schnellen Hochregulation nach pathologischen Prozessen (z.B. mittels Antisense-Technologie); 2. die Inhibierung der enzymatischen Aktivität von 9B5, im Besonderen der Kinase-Aktivität (z.B. mittels eines Kinase-Inhibitors; 3. die Inhibierung einer Interaktion mit einem oder mehreren anderen Molekülen, z.B. downstream befindlichen Protein Kinasen, oder Adaptoren.
In jüngster Zeit wurden spezifische Inhibitoren gegen einige MAP-Protein Kinasen entwickelt. Ein Beispiel dafür ist PD98059, ein Inhibitor von MEK1. Dieser verhindert die Phosphorylierung von Tau durch die Stimulation mit beta-amyloid. Dies hat mögliche Bedeutung für die Therapie der Alzheimer'schen Erkrankung.

Die Antitumorsubstanz UCN-01 ist ein Inhibitor der cdc25c-Phosphorylierung *(*Graves, et al., J Biol Chem, 275, 5600-5, (2000)*).*

Inhibitoren der erfindungsgemäßen Protein-Kinase lassen sich einfach mit herkömmlichen Protein-Kinase-Assays identifizieren und stellen ein wirksames Mittel zum Bekämpfen der Folgeerscheinungen cerebraler Ischämie dar.

Die experimentellen Daten über 9B5 belegen dessen zentrale Beteiligung an Vorgängen, die mit neuronalem Zelltod, Excitation, Plastizität und Neurogenese zusammenhängen. Das erfindungsgemäße Protein stellt ein wichtiges Zielmolekül für die Hemmung bzw. Verringerung der Folgeerscheinungen einer fokalen Ischämie dar. Neben dieser zentralen Rolle bei der Entstehung von Apoplexie als Folge fokaler Ischämie könnte das erfindungsgemäße Protein auch ein wichtiges Zielmolekül bei der Behandlung neoplastischer Erkrankungen, wie Krebs, darstellen. Auch hier könnte das erfindungsgemäße Protein bzw. sein Gen das Zielmolekül für Antikrebsmittel darstellen. Ebenso könnte das Gen eine wichtige Rolle bei Diagnose und Therapie kardiovaskulärer Erkrankungen sein, da es einige gemeinsame Mechanismen bei ischämisch bedingten Krankheiten gibt.

Bei einer besonders bevorzugten erfindungsgemäßen Ausführungsform wird zur Prophylaxe und Therapie des Schlaganfalls der Spiegel bzw. die Aktivität der erfindungsgemäßen Protein-Kinase gesenkt. Dies kann beispielsweise auf der Ebene der Expression erfolgen, indem die Expression/Translation der entsprechenden Nukleinsäuren gehemmt bzw. erniedrigt wird, oder auf der Ebene der Proteinaktivität erfolgen, indem die Protein-Kinase-Aktivität durch geeignete Inhibitoren erniedrigt bzw. gehemmt wird.

Die erfindungsgemäße Nukleinsäure und das hiervon kodierte Protein eröffnen neue Therapieansätze. So kann beispielsweise der Spiegel der endogenen Nukleinsäure beeinflußt werden, indem entweder direkt auf deren Transkription Einfluß genommen wird oder aber deren Translation zu erfindungsgemäßem Protein beeinflußt wird. Hierfür kommen beispielsweise gentherapeutische Ansätze in Frage, bei denen mittels Co-Supression oder Antisensetechnologie die Translation endogener Transkripte erniedrigt wird. Auch bieten sich neue Ansätze auf Ebene der Proteinaktivität, indem das erfindungsgemäße Protein beispielsweise das Zielmolekül von pharmazeutischen Wirkstoffen darstellt. So lassen sich mittels pharmazeutischer Wirkstoffe die Aktivität der erfindungsgemäßen Proteinkinase modifizieren, um so in den Mechanismus der Apoplexie regulierend einzugreifen. Eine Klasse solcher pharmazeutischer Wirkstoffe stellen die oben erwähnten Proteinkinaseinhibitoren dar. Grundsätzlich ermöglicht also die Bereitsstellung der erfindungsgemäßen Nukleinsäure bzw. des erfindungsgemäßen Proteins vollkommen neue Wege der Prophylaxe bzw. Therapie des Schlaganfalls.

Die folgenden Beispiele erläutern die Erfindung:

### BEISPIELE

### Beispiel 1

### Molekulare Klonierung von 9B5

### Herbeiführen des Fadenmodells in Mäusen

Zum Herbeiführen einer fokalen cerebralen Ischämie in c57/bl6 Mäusen wurden 3 Monate alte Mäuse benutzt. Nach Herbeiführen einer Inhalationsnarkose (70% N20, 30% 02, 0,8 - 1 % Halothan) wurde ein 5-0 Prolenefaden (Fa. Ethicon), der mit 0.1 % Poly-L-Lysin beschichtet war, über die A. carotis externa in die A. carotis interna bis zum Abgang der A. cerebri media vorgeschoben. Die richtige Position des Fadens wird durch einen Abfall des Laser-Doppler-Signals (Fa. Perimed) auf 10 - 20% des Ausgangssignals angezeigt. Nach Durchführung dieser Operation und gegebenenfalls Bestimmung zusätzlicher physiologischer Parameter (Blutdruck, Puls, Blutgase, Blutglukose) erwachen die Mäuse aus der Narkose. Nach bestimmten Okklusionszeiten werden die Mäuse wieder einer Narkose unterzogen', und der Faden zurückgezogen. Dadurch findet eine Reperfusion des Gewebes statt. Nach bestimmten Reperfusionszeiten werden die Mäuse durch Genickbruch getötet, und das Gehirn sofort präpariert und auf Trockeneis weggefroren.

Im vorliegenden Fall wurden keine Reperfusionen durchgeführt, sondern nur eine Okklusion von 90 min bzw. 24 h.

### Präparation von mRNA aus den Hirnen

Dazu wurde das mRNA Präparationskit von der Fa. Invitrogen (Fasttrack) verwendet.

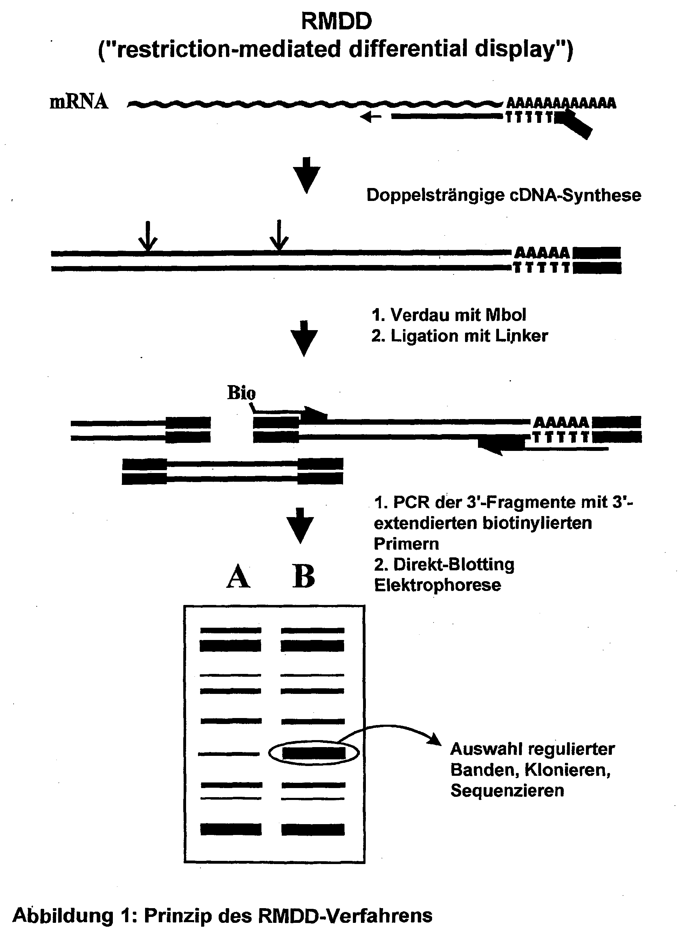

### Durchführung des RMDD Protokolls (siehe auch Fig. 1)

Dabei wurde im wesentlichen nach Pat. Nr. EP 0 743 367 A2; US 5,876,932 verfahren, mit der Änderung, dass 2 µg polyA-RNA für die Erststrangsynthese eingesetzt wurden. Nach Durchführung von Erststrang-, Zweitstrangsynthese, Mbol-Restriktion wird eine Ligation mit Adaptoren durchgeführt. Zwei aufeinanderfolgende PCR-Reaktionen mit Subsets von Primerkombinationen schliessen sich an. Die PCR-Reaktionen werden danach auf ein denaturierendes Polyacrylamid-Gel geladen und auf eine Nylonmembran geblottet (Fa. GATC). Die biotin-markierten Banden werden mit Hilfe einer gebräuchlichen Streptavidin-Peroxidase-Reaktion visualisiert. Auf das Gel wurden jeweils PCR-Proben von der ischämischen und kontralateralen Hemisphäre zusammen aufgetragen (24 h MCAO rechts und links und 90 min MCAO rechts und links). Banden unterschiedlicher Intensität in der rechten oder linken Hemisphäre werden ausgeschnitten, und eine Reamplifikation des entsprechenden PCR-Produktes durchgeführt. Erhaltene amplifizierte Produkte werden in den TOPO TA Vektor pcDNA 2.1 (Fa. Invitrogen) kloniert und mit T7 und M13rev-Primem sequenziert (ABI 3700 Kapillarelektrophoresesequencer).

### Klonierung von 9B5

Bei Durchführung dieses Verfahrens fiel eine Sequenz auf, die nach 90 min auf der ischämischen Seite hochreguliert schien. Diese wurde 9B5 genannt Eine Lightcycler-Analyse bestätigte eine schnelle Regulation nach MCAO (90 min MCAO und 2 h Reperfusion) (Fig. 4).

Das isolierte 3'-gelegene PCR-Fragment wurde benutzt, um eine Maushimbank zu hybridisieren. Dabei fanden sich mehrere Klone, die Sequenzteile von SEQ ID Nr. 1 und SEQ ID Nr. 2 enthielten. Eine Maus Probe aus dem 5'- Bereich wurde benutzt, um eine humane fetale Himbank in lambdaZapll (Stratagene) zu screenen. Dabei ergaben sich aus mehreren Klonen ebenfalls 2 verschiedene Sequenzen, die vermutlich Spleissvarianten desselben Gens darstellen (SEQ ID Nr. 3 und 4). Im Folgenden ist die detaillierte Herstellung der benutzten humanen cDNA-Bibliothek aufgeführt:

### Herstellung der humanen cDNA-Bibliothek

Mit dem cDNA-Synthese Kit der Fa. Stratagene wurde ausgehend von 2 µg humaner fötaler Gehim-mRNA (Fa. Clontech) und von 5 µg mRNA aus adultem Mausgehirn entsprechende cDNA-Bibliotheken hergestellt. Dabei wurde im wesentlichen entsprechend der Angaben des Herstellers verfahren. Zur Synthese der Erststrang cDNA wurde nach den Herstellerangaben ein oligodT-Primer verwendet. Die klonierungs-kompatiblen (E-coRI/Xhol) doppel-strängigen cDNA Fragmente wurden größenselektioniert (nach Herstellerangaben/ Fa. Stratagene) und in den Plasmidvektor pBluescript SKII (Stratagene) ligiert. Die Ligation wurde durch Elektroporation in E.coli (DH10B, Gibco) transformiert und auf LB-Ampizillin Agar-Platten amplifiziert. Die Plasmid-DNA wurde mittels alkalischer Lyse und lonenaustauscher-Chromatographie isoliert (QlAfilter-Kit, Fa. Qiagen).
Die Komplexität an Einzelklonen betrug für die fetale humane Gehirn cDNA-Bank 4 Millionen. Von jeder cDNA-Bank wurden zufallsmäßig 24 Einzelklone nach Insertgrößen analysiert, die eine Größenverteilung von 800 bp bis zu 4.5 kB zeigten, die durchschnittliche Länge der cDNA-Inserts betrug für die humane Bank ca. 1.2 kB.

### Beispiel 2

### Durchführung eines Reportergenassays

Die erhaltene Sequenz von 9B5 kann dazu benutzt werden, Aufschlüsse über die Einordnung des Proteins in Signaltransduktionskaskaden zu gewinnen. Dazu wird das offene Leseraster des Gens in einen gängigen Expressionsvektor (z.B. pCMV-tag, Fa. Stratagene) kloniert. Dieses Konstrukt kann mit anderen Konstrukten zusammen in eukaryontische Zellen transfiziert werden (z.B. mit der Calciumphosphatmethode, siehe Ausubel et al., Current Protocols in Molecular Biology, New York, 1997). Dies können Reporterkonstrukte sein, z.B. ein Luciferasegen unter Kontrolle eines Minimalpromotors mit mehreren Bindungsstellen für z.B. den Transkriptionsfaktor NF-kappaB oder AP-1. Extrakte aus den Zellen können dann einer Messung im Luminometer (z.B. Fa. Bertold) unterzogen werden. Eine Erhöhung des Luciferasewertes deutet auf eine Beeinflussung des Signaltransduktionsweges hin, der in der Aktivierung eines bestimmten Transkriptionsfaktors mündet. Kombinationen mit Expressionskonstrukten für andere Gene (z.B. MAP-Kinasen) können Aufschlüsse über die genaue Position von 9B5 in Signalkaskaden geben. Diese Reporterassays können auch mit anderen Systemen durchgeführt werden, z.B. lacZ oder Chloramphenicoltransferase (CAT-assays), ohne dass das Prinzip des Assays beeinflusst wird.

In derselben Weise können auch vorgefertigte Kits (z.B. Mercury in vivo kinase assay kits, Fa. Clontech) benutzt werden. Dabei wird der Tet-Repressor in Fusion mit der Transaktivatordomäne eines Phosphorylierungstargets (Transkriptionsfaktoren, z.B. Jun) exprimiert. Die Aktivierung eines Luciferasekonstruktes unter Kontrolle eines Tet-Repressor-Elements findet nur statt, wenn eine spezifische Phosphorylierung der Transaktivatordomäne durch eine Kinase (z.B. 9B5) erfolgt. Auf diese Weise ist die Einordnung in einen zellulären Signaltransduktionsweg möglich.

### Beispiel 3

### Kinase-Assays

Protein-Kinasen sind biochemisch sehr gut charakterisiert. Die Kinase-Aktivität von 9B5 kann nachgewiesen werden, indem das offene Leseraster von 9B5 in einen Expressionsvektor mit einem Epitop-Tag kloniert wird (z.B. pcDNA-myc-his), und in eukaryontische Zellen transfiziert wird (z.B. Cos-Zellen). Nach 48 h können Extrakte aus diesen Zellen gewonnen werden und eine Immunpräzipitation mit einem myc-spezifischen Antikörper und ProteinA-Beads durchgeführt werden. In einem Kinasepuffer in Anwesenheit von γ-³²P-ATP wird eine Kinase-Reaktion durchgeführt. Die Proteine werden daraufhin denaturiert und auf einem SDS-PAGE Gel aufgetrennt. Danach wird eine Autoradiographie durchgeführt. Markierte Banden geben Hinweise auf die Kinase-Aktivität von 9B5. Häufig findet sich auch eine Autophosphorylierung der Kinase selbst. Hinweise auf mögliche Phosphorylierungstargets geben auch Kotransfektionen mit potentiellen Targets, z.B. verschiedenen MAP-Kinasen, die ebenfalls mit einem Tag versehen werden und immunpräzipitiert werden können.

Kinase-Assays können auch mit 9B5 durchgeführt werden, das in vitro transkribiert /translatiert wurde (z.B. T7-Reticulozytensystem der Fa. Promega). Ebenfalls kann Protein benutzt werden, das in E.coli exprimiert wurde, z.B. als GST-Fusionsprotein oder als HIS-getagtes Konstrukt. Die Tags können dabei zur Aufreinigung des Proteins benutzt werden. Die gereinigten Proteine können dann in Kinasepuffer mit potentiellen Substraten inkubiert werden. Häufig wird hier MBP (myelin basic protein) verwendet, das häufig von Ser-Thr-Kinasen unspezifisch phosphoryliert wird.

Die Kinasedomäne von Protein-Kinasen ist sehr gut definiert. Häufig genügt die Mutation einzelner Aminosäuren in der Phospatübertragungsdomäne für den Funktionsverlust des Proteins (z. B. K709M bei ASK1 *(*Chang, et al., Science, 281, 1860-3, (1998)*) ;* K90A in DRAK 1, K62A in DRAK2 *(*Sanjo, et al., J Biol Chem, 273, 29066-71, (1998)*)* ; KK429-430AA in NIK *(*Sanjo, et al., J Biol Chem, 273, 29066-71, (1998)*)*; K63W in TAK1 *(*Ninomiya-Tsuji, et al., Nature, 398, 252-6, (1999)*)* ). Diese inaktiven Kinasen sind oft als dominant-negative Inhibitoren für die Evaluation des zellulären Pathways von grosser Bedeutung, z.B. in Kotransfektionsexperimenten und Kinaseassays *(*Ninomiya-Tsuji, et al., Nature, 398, 252-6, (1999)*).* Eine solche Mutation lässt sich auch bei 9B5 durchführen.

Die spezifische Phosphorylierung von Zielprotein lässt sich auch mit Phosphorylierungsspezifischen Antikörpern nachweisen, z.B. Phospho-SerThr/Tyr monoclonal antibody, mouse IgG2b, Fa. Clontech.

Weitere Beispiele für Kinaseassays, die in der Fachwelt gängig sind, sind z.B. dem Buch Protein Phosphorylation, A practical approach, ed. D.G.Hardie, 2nd ed., Oxford, 1999 zu entnehmen, insbesondere den Kapiteln 9 und 10.

### Beispiel 4

### Identifikation des Phosphorylierungstargets von 9B5

Die Identifikation von Phosphorylierungstargets von 9B5 kann zum Beispiel über Interaktionsscreening verlaufen. Dabei können zum einen eher klassische Peptidexpressionsbanken im Bakteriophagen lambda (das meistbenutzte System ist der Vektor lambda-gt11, siehe Ausubel et al., Current protocols in molecular biology, New York, 1997) benutzt werden. Ein Ansatz ist die Klonierung von 9B5 in einen bakteriellen Expressionsvektor unter Einbeziehung eines Aufreinigungstags (z.B. poly-Histidin oder GST) und einer Konsensusphosphorylierungsstelle für Protein-Kinase A (Sequenz RRASV). 9B5 kann damit in Bakterien nach Standardverfahren exprimiert und aufgereinigt werden. Damit kann 9B5 als Fänger mit ³³P oder ³²P durch Inkubation mit Protein-KinaseA markiert werden. Die Expressionsbank kann dann mit dem markierten 9B5 inkubiert werden. Nach Exposition auf Autoradiogramme können positive Klone identifiziert werden und nach Standardverfahren isoliert und sequenziert werden. Dieses Verfahren kann auch auf folgende Weise abgewandelt werden: Die Autophosphorylierungseigenschaft von 9B5 kann ausgenutzt werden, um 9B5 mit ³³P-γATP über einfache Inkubation zu markieren. Das Verfahren kann auch so modifiziert werden, dass die Expressionsbank mit aufgereinigtem 9B5 und ³³P-γATP unter Phopsphorylierungsbedingungen (Phosphorylierungspuffer etc.) inkubiert wird, und exprimierte Peptide aktiv von 9B5 markiert werden. Dieses Verfahren ist allerdings instabiler als das oben beschriebene. Sequenzierte Peptide können zur Erstellung einer Konsensuserkennungs- und -phosphorylierungssequenz benutzt werden. Dies ermöglicht die Auffindung potentieller Substrate über bioinformatische Methoden. Kandidaten können durch Expression und Inkubation mit 9B5 verifiziert werden. Beispiele für erfolgreiche Durchführung dieser Formen des Expressionsscreenings finden sich in *(*Mochly-Rosen and Gordon, Faseb J, 12, 35-42*, (*1998, Blanar and Rutter, Science, 256, 1014-8*, (*1992, Chapline, et al., J Biol Chem, 268, 6858-61*, (*1993, Chapline, et al., J Biol Chem, 271, 6417-22*, (*1996, Kaelin, et al., Cell, 70, 351-64*, (1992,* Songyang, et al., Curr Biol, 4, 973-82, (1994)*).*

Das Phosphorylierungstarget von 9B5 lässt sich auch in einem yeast-two-hybrid screen *(*Fields and Song, Nature, 340, 245-6, (1989)*)* auffinden. So wurde z.B. die Interaktion von Ras und c-Raf (einer Ser-Thr-Kinase) in einem y2h-System entdeckt *(*Fields and Song, Nature, 340, 245-6, (1989)*).* Ebenso ist die Interaktion der Ser-Thr-Kinase SNF1 mit SNF4 geradezu ein Prototyp für das y2h-System *(*Fields and Song, Nature, 340, 245-6, (1989)*).* Im Prinzip äquivalent zu den Hefescreens können auch Säugetiersysteme verwendet werden *(*Fields and Song, Nature, 340, 245-6, (1989)*)*. Für einen y2h-screen wird das offene Leseraster von 9B5 in einen sog. "bait-Vektor" mit der GAL4-Bindungsdomäne kloniert (z.B. pGBT10, Fa. Clontech). Damit kann eine sogenannte "prey-library" in einem Hefestamm nach mehreren gängigen Protokollen auf interagierende Proteine durchsucht werden. Dabei kann es oft nützlich sein, kinase-negative Mutanten einzusetzen, da diese oft stabiler mit den Phosphorylierungstargets interagieren. Serin-Threonin-Kinasen in einem Syntheseweg können durch Adaptermoleküle in räumliche Nähe gebracht werden, um spezifische Phosphorylierungen besser durchführen zu können *(*Chang, et al., Science, 281, 1860-3, (1998)*), (*Yasuda, et al., Mol Cell Biol, 19, 7245-54*, (*1999, Whitmarsh and Davis, Trends Biochem Sci, 23, 481-5*, (1998*, Whitmarsh, et al., Science, 281, 1671-4, (1998)*)* Es ist deshalb auch möglich, über zwei Schritte im yeast-two-hybrid System auf die Phosphorylierungstargets zu stossen, indem man zunächst ein Adapterprotein kloniert, und mit diesem als "bait" das spezifische Zielmolekül findet. Insgesamt können mit dem y2h-System auch Kartierungs-Experimente für Interaktionsdomänen durchgeführt werden.

Es ist ebenfalls möglich, Co-Immunpräzipitationen aus mit 9B5-Expressionsvektoren transfizierten Zellen zu benutzen, um daran bindende Proteine aufzureinigen, und über Proteinsequenzierungsmethoden (z.B. MALDI) die Gene zu identifizieren.

Ebenfalls möglich ist es, nach einer lmmunpräzipitation mit anschliessendem Kinaseassay aus einem Zellextrakt die phosphorylierten Banden aufzureinigen, und diese zu sequenzieren.

Weitere Beispiele für die Identifikation von Protein-Kinase-Substraten, die in der Fachwelt gängig sind, sind z.B. dem Buch Protein Phosphorylation, A practical approach, ed. D.G.Hardie, 2nd ed., Oxford, 1999 zu entnehmen.

### Beispiel 5

### Apoptoseassays

Sehr viele bisher identifizierter Serin-Threonin-Kinasen sind an apoptotischen Prozessen beteiligt, z.B. ASK1 *(*Zhang, et al., Proc Natl Acad Sci U S A, 96, 8511-5, (1999)*), (*Ichijo, et al., Science, 275, 90-4, (1997)*) ,* DRAKs *(*Zhang, et al., Proc Natl Acad Sci U S A, 96, 8511-5, (1999)*) , (*Ichijo, et al., Science, 275, 90-4, (1997)*).* Die Beteiligung von 9B5 an apoptotischen Kaskaden kann weiterhin untersucht werden, indem Expressionskonstrukte mit 9B5 in eukaryontische Zellen transfiziert werden, und danach die Induktion von Apoptose untersucht wird. Dies kann z.B. durch Anfärbung mit Annexin geschehen (Fa. Roche Diagnostics), durch Antikörper, die die aktive Form von Caspase-3 erkennen (Fa. New England Biolabs), oder durch ELISAs, die DNA-Histon-Bruchstücke erkennen (cell-death elisa, Roche Diagnostics). Diese Induktion von Apoptose kann zelltyp-spezifisch sein, deshalb müssen mehrere Zelllinien und primäre Zellen untersucht werden. Die Induktion von Apoptose kann ebenfalls stimulus-spezifisch sein, deshalb können mehrere Stressituationen für die Beantwortung dieser Frage hilfreich sein, z.B. Hitzeschock, Hypoxiebedingungen, Cytokinbehandlungen (z.B. II-1, II-6, TNFalpha), H₂O₂-Behandlung. An Zelltypen kommen mehrere gebräuchliche Linien, z.B. Cos-Zellen, HEK-Zellen, PC12-Zellen, THP-1-Zellen und primäre Zellen wie z.B. Neurone, Astrozyten in Frage, ebenso wie andere immortalisierte und primäre Zellinien nach Bedarf.

### Beispiel 6

### High-throughput screening assays zur Identifikation von Inhibitoren von 9B5

9B5 kann eingesetzt werden, um Inhibitoren der Interaktion mit seinen Interaktionspartnern (z.B. Adaptormolekülen) zu finden. Dies läßt sich z.B. mit dem FRET-System (frequence resonance energy transfer) durchführen, indem 9B5 mit GFP (green fluorescent protein) und sein Interaktionspartner mit BFP (blue fluorescent protein) in Fusion exprimiert und aufgereinigt wird. In einem zellfreien System kann dann die Abnahme der Emission von BFP als Indikator für die Gegenwart eines Inhibitors benutzt werden beim Absuchen komplexer chemischer Banken.

Das Hauptziel bei der therapeutischen Ausnutzung der entdeckten Proteinkinase ist jedoch zunächst die Ausschaltung der Proteinkinasefunktion, da dies am einfachsten durchzuführen ist. Protein-Kinasen bieten sich prinzipiell für die Durchführung von High-Throughput-Assays zur Identifikation von Inhibitoren (small-molecule-inhibitors) der Kinaseeigenschaft des Proteins an, da sich die Enzymeigenschaft selbst leicht als Indikator verwenden lässt (siehe auch Beispiel 3, Kinaseassays).

Eine einfache Implementierung eines HTS-Systems für 9B5 läßt sich nach der Filterassaymethode von Reuter et al. (Reuter et al., Methods in Enzymology, 255: 245 (1995)) durchführen. Dabei benutzt man MBP als unspezifisches Substrat. Dieses wird auf 96-Loch-Platten, die sich für ELISA eignen (z.B. FlashPlates, NEN Life Science Products, oder NUNC) aufgebracht. Zugegeben wird Reaktionspuffer (3x Kinase Reaktions Puffer enthält: 60 mM HEPES (pH 7.5), 30 mM MgAc, 0.15 mM gammaATP, 3 mM DTT, 0.03 mM Na-Orthovanadat). 0.25 µCi 33P-Gamma-ATP und die beschriebene Kinase werden mit einer Konzentration von nicht mehr als 1 µg/ml zugesetzt (eine Titration sollte zunächst durchgeführt werden). In Gegenwart des potentiellen Inhibitors (z.B. small molecules aus einer chemischen Bank) (z.B. 10 µM) wird die Reaktion für 1 h bei 30° inkubiert. Das gesamte Reaktionsvolumen beträgt 100 µl. Die Reaktion wird durch Zugabe von EDTA (pH 7) bis zu einer Endkonzentration von 80 mM gestoppt. Die Proben werden dann zentrifugiert, und 50 µl des Überstandes auf ein p81 Kationenaustauscherpapier (Fa. Whatman) aufgebracht. Die Filter werden dann 3 mal gewaschen in 200 ml 180 mM Phosphorsäure (je 5 min), und einmal in 200 ml 96% Ethanol. Nach Lufttrocknen wird die Radioaktivität der Filter durch Szintillationszählung bestimmt. Stoffe, die die Kinaseaktivität um >= 50% reduzieren (bei 10 µM) fallen durch eine >50%ige Reduktion der Inkorporation auf. Spezifität und Sensitivität der möglichen Inhibitoren werden durch Titration zur Bestimmung der IC50, und durch Substituion anderer Kinasen im Assay bestimmt. Relative Vergleiche der Inhibitionswirkung auf andere Kinasen erlauben so ein Mass für die Spezifität.
Für die Durchführung von Screens auf Kinaseinhibitoren bieten sich auch modernere Systeme mit dem scintillation proximity assay (SPA) an (Fa. Amersham Life Science, MAP kinase SPA; *(*Zhang, et al., Proc Natl Acad Sci U S A, 96, 8511-5, (1999)*)*, *(*Ichijo, et al., Science, 275, 90-4, (1997*))* ). McDonald beschreibt einen Assayaufbau für die Raf/MEK/ERK Kaskade, die Inhibitoren der ganzen Kaskade identifizieren kann. Als Substrat wird hier ein biotinyliertes Peptid benutzt, das nach Phosphorylierung mit ³³P an Avidin-beschichtete SPA-beads binden kann. Die MAP-Kaskade wird hier in vitro rekonstituiert, mit den einzelnen Bestandteilen als GST-Fusionsprotein in *E. coli* exprimiert oder im Falle von cRAF1 mit dem Baculovirussystem hergestellt. Das erste Element der Kaskade (MAP-KKK) muss hierbei ständig gleichmässig aktiviert sein, um verlässlich Inhibitoren screenen zu können. Dies wurde in diesem Fall durch Coexpression von src im Baculovirussystem erreicht. Dadurch wird eine ras-analoge Aktivierung von cRaf erreicht. Denkbar ist auch prinzipiell eine andere Art, eine MAP-Kinase durch Phosphorylierung zu aktivieren. Eine andere Möglichkeit der konstitutiven Aktivierung einer MAP-Kinase besteht in der Mutierung der zu phosphorylierenden Aminosäuren. Dies war im Falle von MEK1 z.B. möglich durch Austausch der Serine Ser218 und Ser222 durch Glutamat *(*Zhang, et al., Proc Natl Acad Sci U S A, 96, 8591-5, (1999*)), (*Ichijo, et al., Science, 275, 90-4, (1997)*).* Im Falle von 9B5 kann man zunächst einen Interaktionsscreen (z.B. y2h-System) durchführen, um ein Phosphorylierungstarget zu identifizieren, oder direkt MBP (myelin basic protein) als Substrat verwenden. Danach kann ein Peptid aus der Zielsequenz mit einem Biotinanker synthetisiert werden. Dieses kann an Avidingekoppelte SPA-beads der Fa. Amersham binden. In die Reaktion wird noch aufgereinigtes (z.B. bakteriell produziertes) 9B5-Protein zugegeben und gamma-32P-ATP. Dies kann im Microtitermasstab erfolgen. Unter Normalbedingungen wird das Ziel-Peptid phosphoryliert und eine Szintillationsantwort auslösen. Vor Zugabe des Ziel-Peptids wird man nun aus einer Bibliothek chemischer Substanzen solche mit der Reaktion vorinkubieren. Nach Zugabe des Ziel-Peptids kann dann die Szinitillationsantwort gemessen werden. Ein Abfall der Antwort bedeutet die Gegenwart eines potentiellen Inhibitors. Die durchsuchte Bibliothek sollte möglichst komplex sein, und viele unterschiedliche Substanzklassen, enthalten. Beispiele für eine Substanzklasse spezifischer Inhibitoren für eine Proteinkinase (p38) finden sich beispielsweise in US Pat Nr. 5,945,418. Andere Substanzklassen die Proteinkinasen inhibieren können sind z.B. bis monocyclische, bicyclische oder heterocyclische Arylverbindungen (WO 92/20642), Vinylen-azaindol Derivate (WO 94/14808), 1-Cyclopropyl-4-pyridyl-quinolone (US-Patent No. 5,330,992), Styrylverbindungen (US Patent No. 5,217,999), styryl-substituierte Pyridyl-Verbindungen (US Patent No. 5,302,606), bestimmte Quinazolin-Derivate (EP-Anmeldung No 0 566 266 A1), Seleoindole und Selenide (WO 94/03427), tricyclische polyhydroxylische Verbindungen (WO 92/21660), und Benzylphosphonische Säuren (WO 91/15495).

Das Spezifische des Assays ist allein bestimmt durch die Identität der beschriebenen Ser-Thr-Kinase. Es können verschiedene Substrate benutzt werden. Diese können in verschiedenen Ausformungen des o.a. Assayprinzips eingesetzt werden. Dabei ist der wesentliche Punkt jedoch immer die Benutzung der Kinaseaktivität von 9B5 zu Screeningzwecken. Beispielsweise kann das Substrat in freier Lösung vorkommen, wie auch an eine Phase gekoppelt sein, wie oben angeführt. Das Substrat kann jedoch auch auf einer Zelloberfläche getragen werden, oder sogar intrazellulär angeboten werden. Dies hat keinen Einfluß auf das zugrundeliegende Assayprinzip

### Beispiel 7

### Herstellung von transgenen und knock-out Mäusen

Die Kenntnis der Sequenz von 9B5 kann dazu benutzt werden, genetisch veränderte Mäuse (oder andere Tiere) herzustellen. Dazu wird z.B. eine konstitutiv inaktive Mutante von 9B5 in transgenen Mäusen exprimiert, z.B. unter einem NSE-Promotor in Neuronen, unter einem MBP-Promotor in Oligodendrozyten etc. Dies sollte einen dominantnegativen Effekt haben, und so eine Inhibition von 9B5 imitieren. Dies kann wertvolle Hinweise auf mögliche pharmakologische Auswirkungen von Inhibitoren in vivo haben. Ebenso kann eine konstitutiv aktive Kinase exprimiert werden.

Ebenso kann die Herstellung von knock-out Tieren Hinweise auf die Auswirkungen von Inhibitoren haben. Die Herstellung dieser genetisch veränderten Mäuse oder anderer Tiere erfolgt nach Standardverfahren, die dem Fachmann bekannt sind.

Die genetisch veränderten Tiere können danach in unterschiedlichen Krankheitsmodellen untersucht werden (z.B. experimentell herbeigeführtem Schlaganfall, MCAO), oder Tumormodellen.

### Beispiel 8

### Proliferationsassays

Viele der bisher bekannten Serin-Threonin-Kinasen sind an neoplastischen Prozessen beteiligt. Die Beteiligung von 9B5 beim Wachstum von Zellen, dem Zellzyklus, der tumorigenen Transformation kann weiter untersucht werden, indem Expressionskonstrukte mit 9B5 in eukaryontische Zellen transfiziert werden, und danach die Induktion von Tumorigenizität untersucht wird, z.B. in einem Soft-Agar-Test *(*Zhang, et al., Proc Natl Acad Sci U S A, 96, 8511-5, (1999)*), (*Ichijo, et al., Science, 275, 90-4, (1997)*).* An Zelltypen kommen mehrere gebräuchliche Linien, z.B. Cos-Zellen, HEK-Zellen, PC12-Zellen, THP-1-Zellen und primäre Zellen, wie z.B. Neurone, Astrozyten in Frage, ebenso wie andere immortalisierte und primäre Zellinien nach Bedarf.

## Patentansprüche

1. Nukleinsäure, die für eine neuronale Serin-Threonin-Protein-Kinase kodiert, ausgewählt aus
a) Nukleinsäure, mit einer der Sequenzen nach SEQ ID Nr. 1-4 und Nukleinsäure, die für ein Protein mit einer Sequenz gemäß einer der SEQ ID Nr. 5-8 kodiert;
b) Nukleinsäure, die für ein Protein kodiert, das mindestens 70 % Identität zu einem Protein gemäß einer der SEQ ID Nr. 5-8 aufweist, und dessen Expression durch fokale cerebrale Ischämie und Reperfusion induziert werden kann.

2. Nukleinsäure nach Anspruch 1, **dadurch gekennzeichnet, dass** sie für eine Proteinsequenz nach einer der SEQ ID Nr. 5-8 kodiert.

3. Nukleinsäure nach einem der Ansprüche 1 bis 2, **dadurch gekennzeichnet, dass** sie eine DNA ist.

4. Konstrukt, enthaltend eine Nukleinsäure, nach einem der Ansprüche 1 bis 3, vorzugsweise unter der Kontrolle von die Expression regulierenden Elementen.

5. Konstrukt, enthaltend eine Nukleinsäure, nach Anspruch 4, **dadurch gekennzeichnet, dass** sich die Nukleinsäure in Antisinnorientierung zu dem regulatorischen Element befindet.

6. Konstrukt nach einem der vorhergehenden Ansprüche, **dadurch gekennzeichnet, dass** es in einem Plasmid vorliegt.

7. Wirtszelle, enthaltend ein Konstrukt nach einem der Ansprüche 4 bis 5.

8. Wirtszelle nach Anspruch 7, **dadurch gekennzeichnet, dass** sie ausgewählt wird aus Bakterien, Hefezellen und Säugerzellen.

9. Isoliertes Säugergewebe-, organ und/oder nicht-menschlicher transgener Säuger, enthaltend ein Konstrukt nach einem der Ansprüche 4 bis 5.

10. Wirtszelle und/oder Säugergewebe und/oder Säugerorgan und/oder nicht-menschlicher transgener Säuger nach einem der vorhergehenden Ansprüche, **dadurch gekennzeichnet, dass** die Nukleinsäure bzw. das Konstrukt in einer Stelle des Genoms integriert ist, die nicht seiner natürlichen Position entspricht.

11. Protein, erhältlich durch Expression einer Nukleinsäure nach einem der Ansprüche 1 bis 3 in einem Expressionssystem, vorzugsweise einer Wirtszelle.

12. Protein, dessen Expression durch fokale cerebrale Ischämie und Reperfusion induziert werden kann, erhältlich durch Expression einer Nukleinsäure nach einem der Ansprüche 1 bis 3 in einem Expressionssystem, vorzugsweise einer Wirtszelle.

13. Protein nach Anspruch 11 oder 12, **dadurch gekennzeichnet, dass** es ausgewählt wird aus einem Protein mit einer der Sequenzen gemäß SEQ ID Nr. 5 bis 8 und Proteinen, die zu einem der genannten Proteine zu mindestens 70 % identisch sind und deren Expression durch fokale cerebrale Ischämie und Reperfusion induziert werden kann.

14. Antikörper, der mit einem Protein nach einem der Ansprüche 11 bis 13 spezifisch reagiert.

15. Diagnosekit, enthaltend eine Nukleinsäure nach einem der Ansprüche 1 bis 3 und/oder ein Konstrukt nach einem der Ansprüche 4 bis 5 und/oder ein Protein nach einem der Ansprüche 11 bis 13 und/oder einen Antikörper nach Anspruch 14.

16. Verfahren zur Diagnose eines Apoplexierisikos, umfassend den Schritt: Bestimmen des Expressionsspiegels einer Nukleinsäure nach einem der Ansprüche 1 bis 3 oder der Menge an Protein nach einem der Ansprüche 11 bis 13 in einer Patientenprobe.

17. Verfahren nach Anspruch 16, **dadurch gekennzeichnet, dass** die Patientenprobe mit einer Nukleinsäure nach einem der Ansprüche 1 bis 3 und/oder einem Antikörper nach Anspruch 14 in Kontakt gebracht wird.

18. Verfahren zum Identifizieren und/oder Isolieren eines Protein-Kinaseinhibitors, **dadurch gekennzeichnet, dass** die Protein-Kinaseaktivität eines Proteins nach einem der Ansprüche 11 bis 13 in Anwesenheit und Abwesenheit der Testsubstanz bestimmt wird und die so als geeignet identifizierte Substanz isoliert wird.

19. Protein-Kinaseassay, **dadurch gekennzeichnet**, das die untersuchte Protein-Kinase ein Protein nach einem der Ansprüche 11 bis 13 umfasst.

20. Pharmazeutische Zusammensetzung, enthaltend eine Nukleinsäure, ein Konstrukt, eine Wirtszelle, Protein und/oder einen Antikörper nach einem der Ansprüche 1 bis 14.

21. Verwendung einer Nukleinsäure, eines Konstrukts, einer Wirtszelle, eines Proteins und/oder eines Antikörpers nach einem der Ansprüche 1 bis 14 zur Herstellung eines Medikaments für die Prophylaxe und/oder Therapie neurologischer Erkrankungen, einschließlich Schlaganfall, Multiple Sklerose, Morbus Parkinson, Amyotrophe Lateralsklerose; Heterodegenerative Ataxien, Morbus Huntington, Neuropathien und Epilepsien und/oder Tumorerkrankungen, insbesondere Karzinome.

## Claims

1. A nucleic acid that encodes for a neuronal serine threonine protein kinase, selected from
a) a nucleic acid with one of the sequences according to SEQ ID NO: 1 to 4 and a nucleic acid that encodes for a protein with a sequence according to one of SEQ ID NO: 5 to 8;
b) a nucleic acid that encodes for a protein that exhibits at least 70% identity to a protein according to one of SEQ ID NO: 5 to 8, and whose expression can be induces by focal cerebral ischaemia and reperfusion.

2. The nucleic acid according to claim 1, **characterised in that** it encodes for a protein sequence according to one of SEQ ID NO: 5 to 8.

3. The nucleic acid according to claims 1 to 2, **characterised in that** it is a DNA.

4. A construct containing a nucleic acid according to any one of claims 1 to 3, preferably, under control of expression-regulating elements.

5. A construct containing a nucleic acid according to claim 4, **characterised in that** the nucleic acid is in antisense orientation to the regulatory element.

6. The construct according to one of the foregoing claims, **characterised in that** it is present in a plasmid.

7. A host cell containing a construct according to claims 4 to 5.

8. The host cell according to claim 7, **characterised in that** it is selected from bacteria, yeast cells and mammalian cells.

9. An isolated mammalian tissue, organ and/or non-human transgenic mammal containing a construct according to any one of claims 4 to 5.

10. The host cell and/or the mammalian tissue and/or the mammalian organ and/or non-human transgenic mammal according to one the foregoing claims, **characterised in that** the nucleic acid or construct is integrated in a position of the genome that does not correspond to its natural position.

11. A protein obtainable by expression of a nucleic acid according to any one of claims 1 to 3 in an expression system, preferably, a host cell.

12. A protein whose expression can be induced by focal cerebral ischaemia and reperfusion, obtainable by expression of a nucleic acid of one of claims 1 to 3, in an expression system, preferably a host cell.

13. The protein according to claim 11 or 12, **characterised in that** it is selected from a protein with one of the sequences according to SEQ ID NOs. 5 to 8 and proteins that are at least 70% identical to one of the said proteins and whose expression can be induced by focal cerebral ischaemia and reperfusion.

14. An antibody that specifically reacts with a protein according to any one of claims 11 to 13.

15. A diagnostic kit containing a nucleic acid according to any one of claims 1 to 3 and/or a construct according to any one of claims 4 to 5 and/or a protein according to any one of claims 11 to 13 and/or an antibody according to claim 14.

16. A method for the diagnosis of a risk of apoplexy comprising the step of determination of the level of expression of a nucleic acid according to any one of claims 1 to 3 or the quantity of protein according to any one of claims 11 to 13 in a patient sample.

17. The method according to claim 16, **characterised in that** the patient sample is brought into contact with a nucleic acid according to any one of claims 1 to 3 and/or an antibody according to claim 14.

18. A method for the identification and/or isolation of a protein kinase inhibitor, **characterised in that** the protein kinase activity of a protein according to any one of claims 11 to 13 is determined in the presence and absence of the test substance and the substance thus identified is isolated.

19. A protein kinase assay, **characterised in that** the protein kinase investigated comprises a protein according to any one of claims 11 to 13.

20. A pharmaceutical composition containing a nucleic acid, a construct, a host cell, a protein and/or an antibody according to any one of claims 1 to 14.

21. Use of a nucleic acid, a construct, a host cell, a protein and/or an antibody according to any one of claims 1 to 14 for the prophylaxis and/or therapy of neurological diseases, including stroke, multiple sclerosis, Parkinsons's disease, amyotropic lateral sclerosis, heterodegenerative ataxias, Huntington's disease, neuropathies and epilepsies and/or tumor diseases, particularly carcinomas.

## Revendications

1. Acide nucléique, qui code une protéine kinase sérine thréonine neuronale, choisi parmi
a) l'acide nucléique ayant une des séquences d'après les SEQ ID N°1-4 et l'acide nucléique, qui code une protéine ayant une séquence selon l'une des SEQ ID N°5-8 ;
b) l'acide nucléique qui code une protéine, qui présente une identité d'au moins 70 % avec une protéine selon l'une des SEQ ID N°5-8, et dont l'expression peut être induite par une ischémie et reperfusion cérébrale focale.

2. Acide nucléique selon la revendication 1, **caractérisé en ce qu'**il code une séquence de protéine selon l'une des SEQ ID N°5-8.

3. Acide nucléique selon l'une des revendications 1 à 2, **caractérisé en ce que** c'est un ADN.

4. Hybride, contenant un acide nucléique, selon l'une des revendications 1 à 3, de préférence sous le contrôle d'éléments régulateurs de l'expression.

5. Hybride, contenant un acide nucléique, selon la revendication 4, **caractérisé en ce que** l'acide nucléique se trouve en orientation antisens par rapport à l'élément régulateur.

6. Hybride selon l'une des revendications précé-dentes, **caractérisé en ce qu'**il se présente dans un plasmide.

7. Cellule hôte, contenant un hybride selon l'une des revendications 4 à 5.

8. Cellule hôte selon la revendication 7, carac-térisée en ce qu'elle est choisie parmi les bactéries, les cellules de levures et les cellules de mammifères.

9. Tissu, organe isolé de mammifère et/ou de mammifère transgénique non humain, contenant un hybride selon l'une des revendications 4 à 5.

10. Cellule hôte et/ou tissu de mammifère et/ou organe de mammifère et/ou de mammifère trans-génique non humain selon l'une des revendications précédentes, **caractérisé en ce que** l'acide nucléique ou l'hybride est intégré dans un endroit du génome qui ne correspond pas à sa position naturelle.

11. Protéine, pouvant être obtenue par l'expression d'un acide nucléique selon l'une des revendica-tions 1 à 3 dans un système d'expression, de préférence une cellule hôte.

12. Protéine, dont l'expression peut être induite par une ischémie et une reperfusion cérébrale focale, pouvant être obtenue par l'expression d'un acide nucléique selon l'une des revendications 1 à 3 dans un système d'ex-pression, de préférence une cellule hôte.

13. Protéine selon la revendication 11 ou 12, **caractérisée en ce qu'**elle est choisie parmi une protéine ayant une des séquences selon la SEQ ID N°5 à 8 et des protéines, qui sont identiques à une des protéines citées à au moins 70 % et dont l'expression peut être induite par une ischémie et une reperfusion cérébrale focale.

14. Anticorps qui réagit spécifiquement avec une protéine selon l'une des revendications 11 à 13.

15. Kit diagnostic, contenant un acide nucléique selon l'une des revendications 1 à 3 et/ou un hybride selon l'une des revendications 4 à 5 et/ou une protéine selon l'une des revendica-tions 11 à 13 et/ou un anticorps selon la revendication 14.

16. Procédé pour le diagnostic d'un risque d'apoplexie, comprenant l'étape consistant à : déterminer le taux d'expression d'un acide nucléique selon l'une des revendications 1 à 3 ou la quantité de protéine selon l'une des revendications 11 à 13 dans un échantillon prélevé chez un patient.

17. Procédé selon la revendication 16, caracté-risé en ce que l'échantillon prélevé chez un patient est mis en contact avec un acide nucléique selon l'une des revendications 1 à 3 et/ou un anticorps selon la revendication 14.

18. Procédé pour identifier et/ou isoler un inhibiteur de protéine kinase, **caractérisé en ce que** l'activité de protéine kinase d'une protéine selon l'une des revendications 11 à 13 est déterminée en la présence ou en l'absence de la substance test et la substance ainsi identifiée de manière appropriée est isolée.

19. Dosage de protéine kinase, **caractérisé en ce que** la protéine kinase analysée comprend une protéine selon l'une des revendications 11 à 13.

20. Composition pharmaceutique, contenant un acide nucléique, un hybride, une cellule hôte, une protéine et/ou un anticorps selon l'une des revendications 1 à 14.

21. Utilisation d'un acide nucléique, d'un hybride, d'une cellule hôte, d'une protéine et/ou d'un anticorps selon l'une des revendications 1 à 14 pour la fabrication d'un médicament destiné à la prophylaxie et/ou la thérapie des maladies neurologiques, y compris l'attaque, la sclérose en plaques, la maladie de Parkinson, la sclérose latérale amyotrophique, les ataxies hétérodégénératives, la maladie de Huntington, les neuropathies, et les épilepsies et/ou affec-tions tumorales, en particulier les carcinomes.
